(19) Europäisches Patentamt
European Patent Office
Office européen des brevets

(11) **EP 4 194 643 A2**

(12) **EUROPEAN PATENT APPLICATION**

(43) Date of publication:
**14.06.2023  Bulletin 2023/24**

(21) Application number: **22212067.7**

(22) Date of filing: **07.12.2022**

(51) International Patent Classification (IPC):
*E04H 4/12* (2006.01)    *C02F 103/42* (2006.01)

(52) Cooperative Patent Classification (CPC):
**E04H 4/12;** C02F 2103/42; C02F 2209/005

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **08.12.2021  US 202163287183 P**

(71) Applicant: **Maytronics Ltd.
1935000 Kibbutz Yizrael (IL)**

(72) Inventor: **TZUR, Doron
4644637 Herzliya (IL)**

(74) Representative: **Jaeger, Michael David
Withers & Rogers LLP
2 London Bridge
London SE1 9RA (GB)**

(54)  **AN APPARATUS FOR MEASURING CHARACTERISTICS OF A WATER FACILITY**

(57)  Methods, systems and apparatuses using: (i) a flow control unit including: piping inlets and outlets for connecting thereby to different parts located at different locations of a water facility (WF) and of the apparatuses themselves and multiple controllable valves; (ii) a detection unit including at least one sensor for sensing characteristics of the WF in various locations and/or states of the WF; and a controller, configured to control operation of the valves, for measuring one or more characteristics of a selected detection location or state of the WF or of water manipulated within the apparatuses. The methods, systems and apparatuses may further be configured to apply manipulations over a water sample, sampled from the WF and/or over water from the WF and measure responses to the applied manipulation(s).

FIG. 1

EP 4 194 643 A2

## Description

### FIELD OF THE INVENTION

The present disclosure relates in general to systems and methods for measuring physical characteristics of a water facility such as a swimming pool.

### BACKGROUND

Managing maintenance of large water facilities such as swimming pools requires ongoing monitoring and controlling of various parameters indicative of functioning of various parts of the water facility such as the state/functionality of the water facility's piping, pump and/or filter, water characteristics such as polluting and disinfecting materials' concentration, water temperature, water turbidity level, ORP, Ph, alkaline, presence of overly high chlorine concentration levels (due to over-adding of chlorine for disinfection), etc.

### BRIEF DESCRIPTION OF THE FIGURES

The figures illustrate generally, by way of example, but not by way of limitation, various embodiments discussed in the present document.

For simplicity and clarity of illustration, elements shown in the figures have not necessarily been drawn to scale. For example, the dimensions of some of the elements may be exaggerated relative to other elements for clarity of presentation. Furthermore, reference numerals may be repeated among the figures to indicate corresponding or analogous elements. References to previously presented elements are implied without necessarily further citing the drawing or description in which they appear. The figures are listed below.

**Fig. 1** shows main components of an apparatus for measuring characteristics of a water facility, according to some embodiments;

**Figures 2A-2B** show various views of a detection unit of the apparatus or part(s) thereof, according to some embodiments: **Fig. 2A** shows an exploded view of the detection unit including a sampling module and three optical modules; and **Fig. 2B** shows the sampling module of the detection unit;

**Fig. 3** shows a packaging unit for encasing and optionally also water-proofing the detection unit modules among other parts of the apparatus leaving only portions of the inlets and outlets thereof extending outwardly from the packaging unit for channeling the water therethrough;

**Figures 4A-4B** shows a packaging unit for the apparatus that includes a cooling mechanism on one side thereof: **Fig. 4A** shows the apparatus having encased by the packaging unit with the cooling mechanism disposed at one side therefor; **Fig. 4B** shows the cooling mechanism in a zoomed-in view; and **Fig. 4C** shows a cooling channel of the cooling mechanism in a zoomed in view;

**Fig. 5** shows optical modules of an apparatus for measuring characteristics of a water facility having a curved optical waveguide, according to some embodiments;

**Fig. 6** shows an apparatus for measuring characteristics of a water facility connecting to various piping areas of a piping of a water facility (WF), according to some embodiments;

**Fig. 7** is a block diagram, shows a system for measuring characteristics of a swimming pool water facility using a single pressure gauge hydraulically and switchably connected to a piping manifold of the swimming pool, according to some embodiments;

**Fig. 8** shows a list of solenoid valves states combination for various treatments and/or measurements acquisition purposes, for the system outlet shown in **Fig. 7,** according to some embodiments;

**Fig. 9** is a block diagram, showing valves and piping connections of a system for measuring characteristics of a water facility, according to some embodiments;

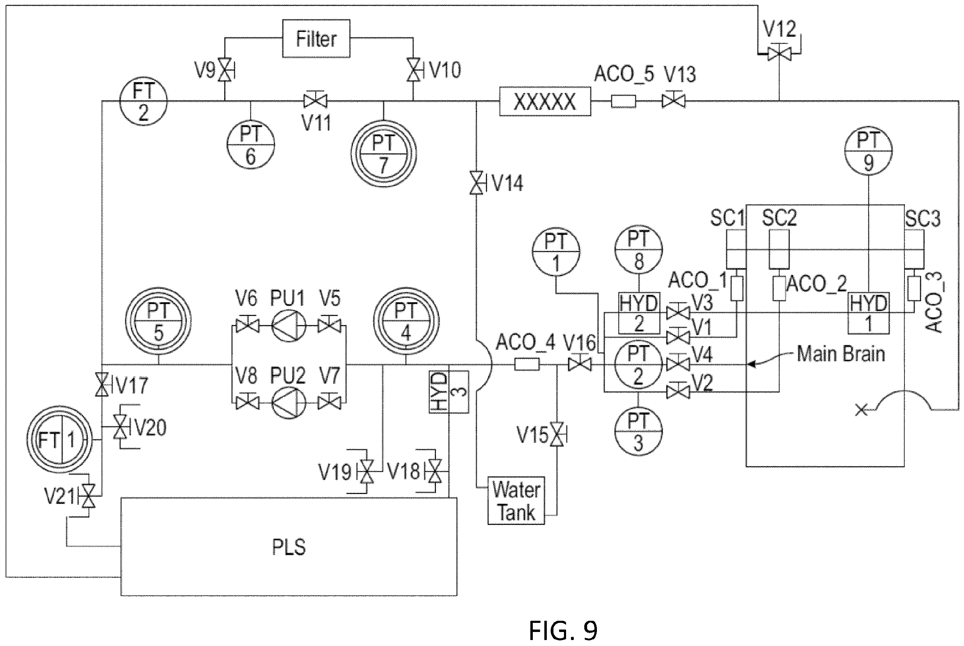

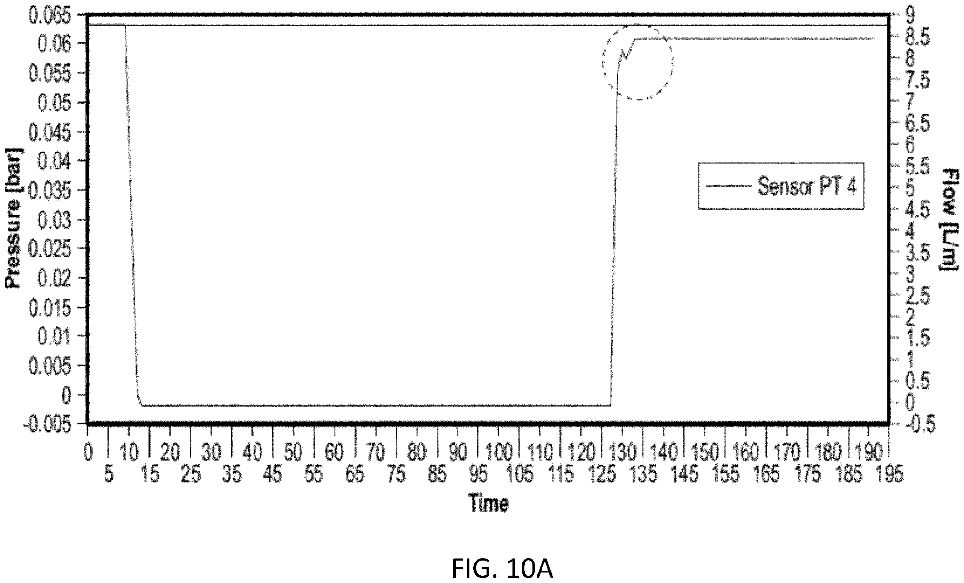

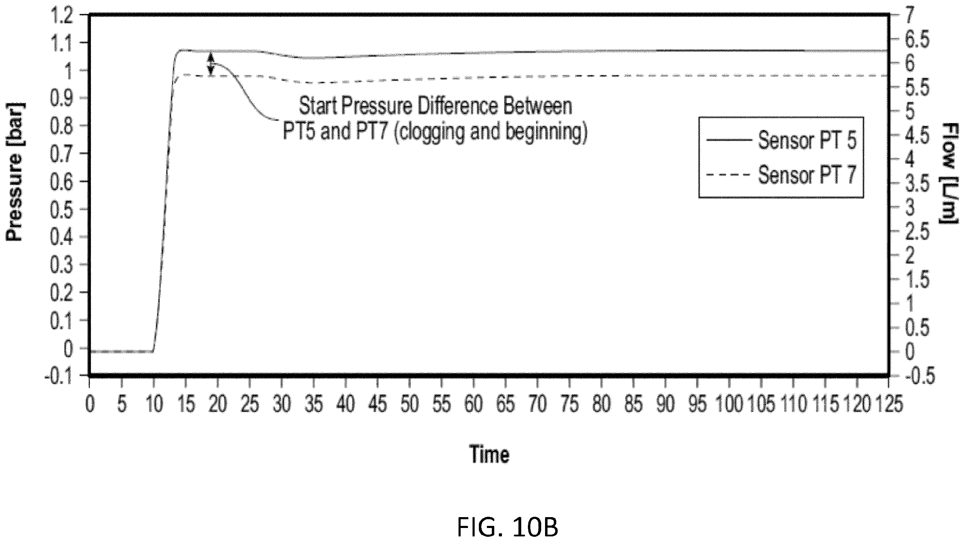

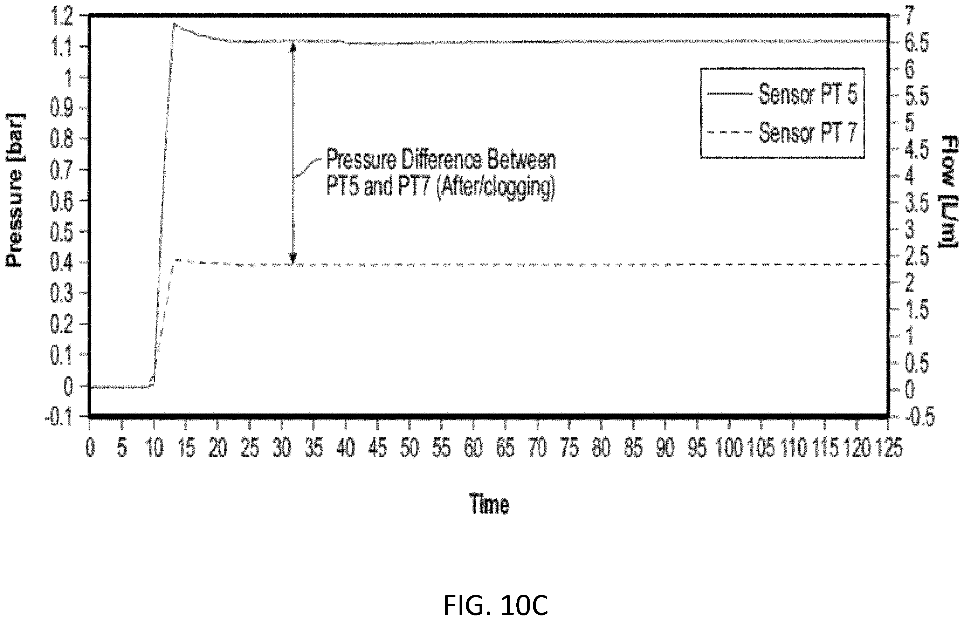

**Figures 10A-10C** show outputs behavior of various pressure sensors, located and configured to measure the WF in various locations in different states of the measured WF location, according to some embodiments, using the system layout of **Fig. 9: Fig. 10A** shows exemplary output of sensor PT4 for measuring level of the water in a swimming pool WF; **Fig. 10B** shows exemplary outputs of sensors PT5 and PT7 for measuring clogging by measuring differences between two sensors' output, where the sensors are located at different strategic locations; **Fig. 10C** how the clogging of the WF area is manifested in an enlarged difference between the sensors' outputs;

**Fig. 11** shows differences in pressure value behavior for clogged and unclogged pump-filter that can be used to detect clogging impairments in the WF by use of one or more pressure sensors connected such as to measure piping pressure of pipes sections located before and after the filter pump, according to some embodiments; and

**Fig. 12** shows a flowchart illustrating a method measuring characteristics of a water facility, according to some embodiments.

## DETAILED DESCRIPTION

Aspects of disclosed embodiments pertain to apparatuses, systems and methods for monitoring various characteristics of one or more water facilities, indicative of functionality of various parts, components and/or properties of each water facility (WF) such as characteristics of the water in the WF, WF piping condition and location of WF piping-related impairments, condition of WF working/maintenance devices such as pump(s), filter(s), water-disinfectant(s) dispensers, etc.

The methods, systems and apparatuses, according to some embodiments of the invention, may further be configured to apply one or more treatments/manipulations to water of the WF and/or to at least one water sample acquired from the WF, and measure WF characteristics by measuring responses to the applied treatment(s)/manipulation(s) over the water sample and/or the WF.

The term "water facility" (WF) may relate to any facility that enables containing, channeling and/or treatment of water or any other liquid used in large quantities, such as a swimming pool, a water/liquid reservoir, water/liquid channeling piping system, etc.

According to some embodiments, the apparatuses and systems may be designed to removably connect to various parts of the WF via multiple connecting ports such as inlet and outlet pipes, and valves such as electronically controllable valves (e.g. solenoid valves shortly referred to herein as solenoids), and to selectively control operation of the valves in order to control measurements of various parts of the WF as well as to control measuring of parts of the WF in different states of the same respective part.

For example, the apparatus may be configured to measure various parts of the WF before and after various treatments applied to a sample of the WF water by acquiring a water sample from different parts of the WF, such as, for instance, acquiring water samples arriving to the WF filter (pre-filtering state) and exiting the filter (post-filtering state).

Aspects of disclosed embodiments pertain to an apparatus for measuring characteristics of a WF including characteristics of the WF piping, WF devices' functioning such as filter(s)/pump(s) functionality, water characteristics such as concentration of substances (polluting chemical and/or biological substances), water flow and water pressure, level, volume or quantity of water in a water reservoir of the WF etc., using at least:

a flow control unit including at least: multiple piping inlets and outlets connecting to different parts located at different locations of the WF piping setup; and multiple controllable valves, each of the controllable valves being deployed at a different inlet or outlet of the multiple piping inlets and outlets, such as to control water flow to and from different parts of the WF, located at different locations of the WF piping setup;

a detection unit comprising at least one sensor or detector such as at least one of: a pressure sensor, an optical detector such as spectrometer, an acoustic sensor/transducer, a thermometer, etc.; and

a main processing and control unit (MPCU), operatively associated with the flow control unit and with the detection unit.

The MPCU and the flow unit may be configured to control operation of the controllable valves by selecting one of multiple valves states combinations (VSCs), each VSC being associated with a different location and/or a different manipulation of a specific part of the WF and/or of the water sample.

According to some embodiments, the detection unit may further include: a sampling module including a water-sample holder (herein also "sample holder") configured for holding therein a water sample from the WF. The detection unit may include one or more optical detectors such as a spectrometer for measuring optical characteristics (e.g., spectrum/absorption characteristics) of the water sample . The detection unit may include one or more optical modules, the optical modules being configured and located to optically detect characteristics of the water sample in various pre/post treatment states of the water sample.

According to some embodiments, the MPCU may select locations for measurements, duration and timing of the acquisition according to a predetermined and optionally reprogrammable scheduled monitoring plan. The monitoring plan may include, for example, the scheduled times for measuring pre and/or post treatments of the water such as pre/post filtering, backwash, pumping, chlorination etc. and the order of the treatments being checked, e.g., by selecting a valves states combination (VSC) that is associated with the desired check and associated (coordinated) measuring equipment operation, acquisition duration, and corresponding control and operation commands associated therewith (for switchable closing and opening of each valve based on the selected VSC).

According to some embodiments, the sample holder may include a cuvette which is a fully or partially transparent liquid-container, for enabling optical (e.g., spectrometric) measuring of characteristics of water/liquid contained in the cuvette and optionally also irradiation of the water/liquid in the cuvette.

According to some embodiments, the sample holder may further include a casing partially covering the cuvette for allowing light to be passed through specific sides of the cuvette e.g. for allowing light to imping the water sample, from one or more light sources arriving from one or more directions and sides of the cuvette, and to allow light passed through the water sample and possibly reflected, deflected, fluorescence, scattered and/or diffracted by the water sample and/or by the cuvette to be directed towards one or more optical detectors located at one or more different sides in respect to the cuvette.

According to some embodiments, the apparatus may be configured for using water samples from different WF parts/locations by selecting a different "detection location" of the WF, for example, by using means for acquiring water sample of each WF part/location into the cuvette (e.g. by receiving a water sample into the cuvette via suction and/or pumping of water therein from a different piping inlet associated with the respective WF part/location) and optionally drainage of the acquired water sample for emptying the cuvette to enable acquisition of another water sample from a different part/location of the WF (e.g. using one or more drainage outlets of the flow control unit). Alternatively, to replace the water sample, a different water source may be directed into the cuvette (without draining of the sample already existing in the cuvette and waiting a predefined time period for the water composition to stabilize in the cuvette.

According to some embodiments, the piping inlets and outlets of the flow control unit may connect to the sample holder (e.g. cuvette) for example, via a filling port connected to all inlets directing water into the cuvette for filling thereof, and a drainage port for enabling drainage of the water sample for acquiring different samples from different WF locations and/or samples of pre and/or post various treatments of the water sample and/or of the WF water. The water sample can be replaced by enabling channeling water from at least some of the connectable part of the WF into the sample holder and drainage of water from the sample holder. In this way all or most of the measurements of the different WF locations and states (pre/post treatments) may be measured by measuring water samples acquired from the respective location with which each treatment state is associated.

According to some embodiments, each detection location may be associated with a selected combination of closed and open valves (valves states combination (VSC)) that enables detection of water characteristics of a specific location/part of the WF or specifically in a certain location of a piping of the WF. In this way the MPCU may be configured to select a detection location of the WF by selecting a VSC that is associated with each WF location selection and control the valves and the acquisition and drainage means based on the required detection location and its associated VSC.

According to some embodiments, the apparatus may be further configured to measure characteristics of the water sample in other pre and/or post treatments states by applying a treatment directly to the water sample in the cuvette (herein "sample treatment") and measuring characteristics of the water sample before and after the application of the respective sample treatment. For example, to measure water turbidity and/or water hardness, an acoustic sample treatment may be applied to the water sample via one or more acoustics transducers, such as acoustic transducers operating in the ultrasonic (US) acoustic range, where the characteristics of the water sample may be measured before and after the acoustic sample treatment using optical and/or acoustical detectors or sensors. The acoustic transducer(s) may be placed in proximity to or in engagement with the cuvette's outer walls.

According to some embodiments, the apparatus may be further configured to divert water towards separate manipulation areas for application of one or more treatment/manipulation/examination/measuring actions over the diverted water (such as de-chlorination, microbial incubation etc.). After completion of the treatment in some cases, the system may be also configured to channel the treated water back into the WF container or back to the cuvette for further measurements and analysis.

According to some embodiments, the apparatus may be part of the WF.

According to other embodiments, the apparatus may be packaged and designed such as to enable easy removable installment thereof, optionally requiring some permanent adjustments made to the specific WF such as customized connectors that can connect the various parts of the specific WF piping to inlets/outlets of the flow control unit and/or of the detection unit.

For example, the apparatus may include a housing enabling inlet and outlet pipes to extend from an external side of the housing for connecting the apparatus to different parts of the WF piping. The entire package of apparatus may be easily connected to the WF and easily removed therefrom, e.g. for replacement thereof, by disconnecting and reconnecting from and to the inlets and outlets of the apparatus via connectors

According to some embodiments, the WF may be designed such as to allow such easy disconnecting and reconnecting with the apparatus, e.g. by having required piping channels thereof extending from different parts thereof, such as to connect with inlets/outlets of the apparatus, optionally via designated valves/piping connectors.

According to some embodiments, the piping of the WF, in which the systems and methods disclosed are embedded or connected to, may include a main pipeline connecting between a main water reservoir of the WF (e.g. the water-pool of the swimming pool WF) and WF maintenance, treatment, monitoring and/or water-supply systems, devices and/or facilities such as the WF main pump(s) and filter(s), one or more drainage channeling means, water faucet(s) for supplying/filling water to the WF, and the like.

According to some embodiments, the apparatus may be installed on/in or embedded in a water pool related platform, device, element and/or system such as in a robotic cleaner machine or a buoy.

According to some embodiments, the apparatus may further include a cooling mechanism, for cooling the detection unit or part(s) thereof.

The different treatments that can be used for testing WF characteristics may be selectable from a list of optional water sample and/or WF water treatments and/or, manipulations, or action for example, one or more from the list of:

biological and/or chemical;
filtering;
backwash treatment performed at the filter of the WF;
degasification (e.g. debubbling);
alkalinity manipulation;
cooling or heating of the WF water;
water drainage;
application of acoustic (e.g. ultrasonic) signals to the water sample;
chemical treatment to the water sample such as chlorination and/or dechlorinating, other type(s) od disinfection, acidification, basification etc.
accelerated growth of algae or any other one or more biomass types.

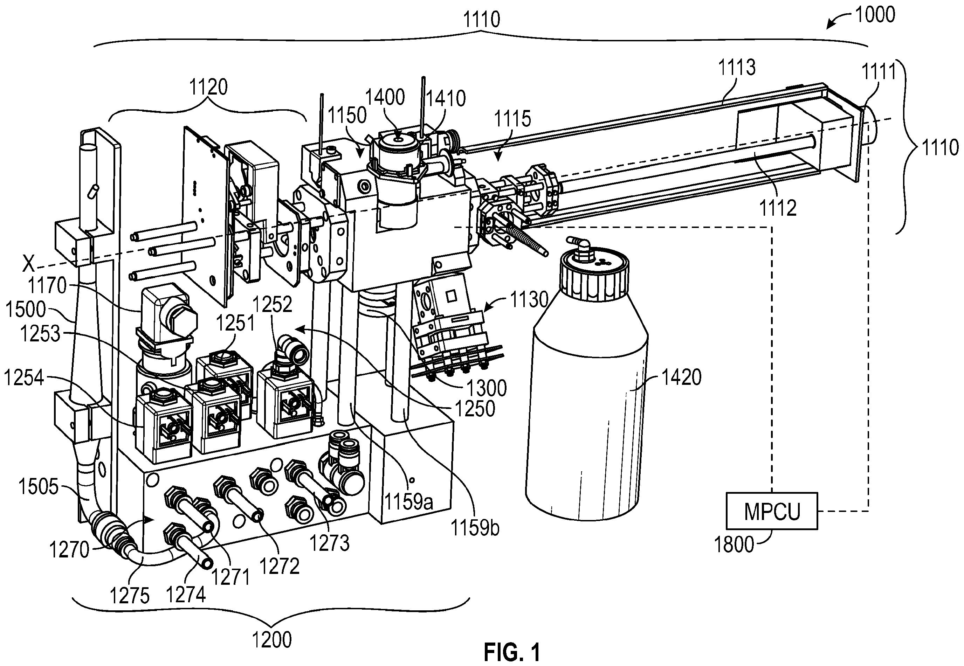

Reference is now made to **Figures 1, 2A, 2B** and **3,** showing an apparatus **1000** for a WF such as a swimming pool or parts thereof, according to some embodiments. The apparatus **1000** may be configured to connect to various parts of the WF via the water piping of the respective WF for measuring various characteristics of the WF in various states of at least some of the WF parts such as pre and/or post one or more treatments of the water running through the piping and/or through the apparatus.

According to embodiments, as shown in **Fig. 1,** the apparatus **1000** includes:

a detection unit **1100** including: (i) at least one pressure sensor such as pressure gauge **1170;** (ii) a sampling module **1150** e.g. including a water-sample holder such as a cuvette **1151** for containing therein a water sample from the WF, (iii) a first optical module **1110** including a spectrometry device **1111,** (iv) a second optical module **1120** and (v) a third optical module **1130,** the detection unit **1100** being configured to detect characteristics of the water sample, using at least one detection technique such as at least one optical detection technique (e.g. using at least one spectrometry-based measure of the water sample);

a flow control unit **1200** comprising a valves set **1250** including multiple controllable valves such as solenoid valves **1251-1254,** and a branching set **1270** including multiple inlets and outlets, such as inlets **1271** and **1272** and outlets **1273** and **1274** for connecting to various parts of the WF piping, the valves **1251-1254** being configured to control water flow through the inlets and outlets **1271-1275,** e.g. where each inlet/outlet has a different controllable valve associated therewith;

a main processing and control unit (MPCU) **1800,** to control the flow control unit **1200** and the detection unit **1100,** and also to receive acquired data from the detection unit **1100,** analyze the acquired data and determine various WF characteristics, based on the data analysis, for determining condition of one or more parts or features of the WF at various locations over the WF such as at various locations over the WF piping as well as to determine WF water condition/quality characteristics such as biomass and/or chemical pollutants' concentration/quantities, water hardness, etc.;

an alkalinity dosing module **1400** including a peristaltic pump **1410** and a container **1420** containing titrant such as an alkali material therein, such as NaOH (sodium hydroxide), the container **1420** connects to the peristaltic pump **1410,** for dispensing of the alkali material into the water sample in the cuvette **1151** e.g. for pH level measurements of the water sample pre and/or post alkalinity dosing (where the term alkalinity or alkali material may include any dissolving material whether having a base pH or an acid pH);

and optionally also:

a degasification module **1500** (de-bubbler) for separating gasses from the water sample in the cuvette (e.g. upon filling of each new water sample in the cuvette and before acquisition of any measurement of the newly filled water sample); and

a cleaning module **1300** configured for cleaning the cuvette **1151** and/or the water sample therein e.g. by using acoustic (e.g., ultrasonic (US)) cuvette-treatment, the cleaning module **1300** may include one or more acoustic (e.g. US) transducers such as US transducer **1301** for applying acoustic stimuli (energy) to the water sample in the cuvette **1151** for cleaning thereof (e.g. by promoting/causing detachment of biological and/or chemical substances from the cuvette inner walls such as algae or microalgae biomass).

According to some embodiments, the MPCU **1800** may select locations for measurements and timing of the acquisition according to a predetermined and optionally reprogrammable scheduled monitoring plan. The monitoring plan may include, for example, the scheduled times for measuring pre and/or post treatments and the order of the treatments being checked, e.g., by having scheduled valves states combination and scheduled (coordinated) measuring equipment operation and corresponding control and operation commands associated therewith (for closing an opening each valve based on the set combination and operating measuring equipment operation etc.).

According to some embodiments the monitoring plan may be adjusted based on real time analysis results for example, for enabling adapting the schedule (acquisition times, cuvette drainage and filling times etc.) to the actual performances of the apparatus **1000.**

According to some embodiments, the MPCU **1800** may be further configured to generate information indicative of analysis results and send the generated information to one or more external devices for displaying the information, further processing of the information, storing the information and the like, using one or more communication technologies, links, networks and/or protocols.

According to some embodiments, the detection unit **1100** may include a flow sensor for measuring the flow in one or more specific junctions of the piping pf the WF such as an acoustic flow sensor (using one or more ultrasonic transducers) or a water system sensor (WSS) such as a pressure sensor, for measuring one or more characteristics of the WF such as pressure and flow rate in parts of the WF piping, pump and/or backwash state etc.

According to some embodiments, the degasification module **1500** may be connected to the flow control unit **1200** via a connecting tube **1505,** for enabling water from the WF piping to be passed through the degasification module **1500** for degasification (debubbling) thereof.

According to some embodiments, the first optical module **1110** may include the spectrometry device **1111,** an optical setup **1115** for directing and optionally focusing or collimating light emanating from the cuvette **1151** to an optical fiber encased by a fiber guide cable **1112,** which guides the light to the spectrometry device **1111.**

According to some embodiments, as shown in **Fig. 1,** the optical setup **1115,** the optical fiber and the spectrometry device **1111** may be arranged in a straight alignment along an alignment axis **x** held via a fixture **1113.**

According to some embodiments, the first optical module **1110** may further include data processing, data storage and/or data communication modules for transmitting and/or processing spectral data outputted by the spectrometry device **1111.** For example, the spectrometry device **1111** may include a low-cost and/or miniature spectrometer.

According to some embodiments, the light arriving at the spectrometry device **1111** may emanate from one or more light sources of the second optical module **1120** and/or from the third optical module **1130** in a switchable and controllable manner, as will be detailed below, such as to pass through the cuvette **1151,** located between each of the second and third optical modules **1120** and **1130** and the first optical module **1110.**

According to some embodiments, as shown in **Fig. 1,** the third optical module **1130** is located angularly to axis **x** forming a non-zero angle (e.g. between 10 to 90 degrees) from a light source(s) thereof to axis **x.** This may enable irradiating the cuvette **1151** from a different angle, using light within the ultraviolet (UV) and/or visible (VIS) range that causes scattering and/or fluorescence of the water sample, e.g. for measuring turbidity level, hardness and/or biomass concentration of the water sample.

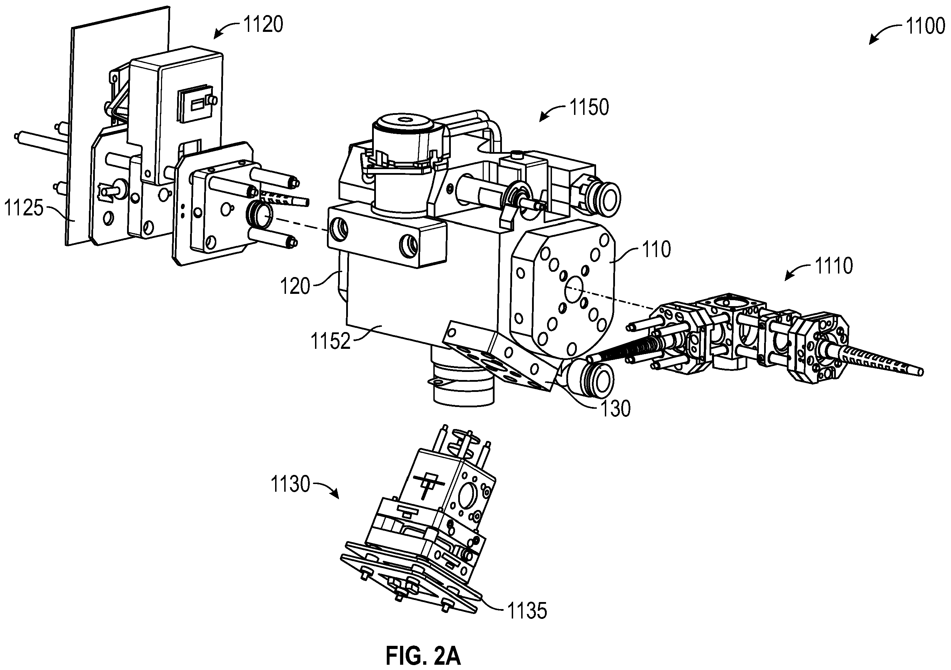

According to some embodiments, as shown in more details in **Figures 2A-2B,** the sampling module **1150** includes the cuvette **1151** and a casing **1152** having several windows such as window **1158** for allowing light to enter and exit the cuvette **1151** from light sources of the optical modules **1120** and **1130** and towards optical detectors located in either one of the optical modules **1110-1130.**

According to some embodiments, as shown in more details in **Figures 2A-2B,** each optical module of optical modules **1110, 1120** and **1130** may be coupled to a different side of the cuvette casing **1152** via a corresponding custom-made plate: **110, 120** and **130,** respectively. Each plate of the plates **110, 120** and **130** may have at least two transparent windows respectively, (e.g. quartz window or an opening) for allowing light to pass therethrough.

According to some embodiments, one or more of the optical modules may include at least one optical detector and a processing unit such as a printed circuit board (PCB) such as PCBs **1125** and **1135** of the second and third optical modules **1120** and **1130,** respectively. The second and third optical modules **1120** and **1130** may further include one or more light sources (not shown), e.g. emitting light in one or more wavelengths or wavelength ranges (for example within the visible (VIS) or ultraviolet (UV) range), for enable measuring different characteristics of the water sample as well as to enable light sources inspection and adjustment/calibration of analysis module(s) used for analyzing optical detectors output data for detection of water sample characteristics and therefore for detection of WF characteristics/functioning.

The apparatus **1000** may further include one or more additional detectors or sensors such as one or more of: a thermometer (e.g. for measuring water temperature in the cuvette **1151),** a conductivity sensor (e.g. for measuring conductivity of the water sample in the cuvette **1151),** acoustic transducer(s) affording for spectrometric optical measurements of pre/post acoustic treatment of the water sample, ultrasonic water flow rate measurements (e.g. by measuring speed-of-sound between transducers), optical and/or acoustic temperature measurements, etc.

For example, two US transducers **1101** and **1102** may be located at opposite sides of the cuvette casing **1152** configured for any one or more of: measuring of flow rate, or water temperature based on the speed of sound. US transducer **1300** may be used for US water treatment, measurement of water hardness via changes of turbidity level following US treatment.

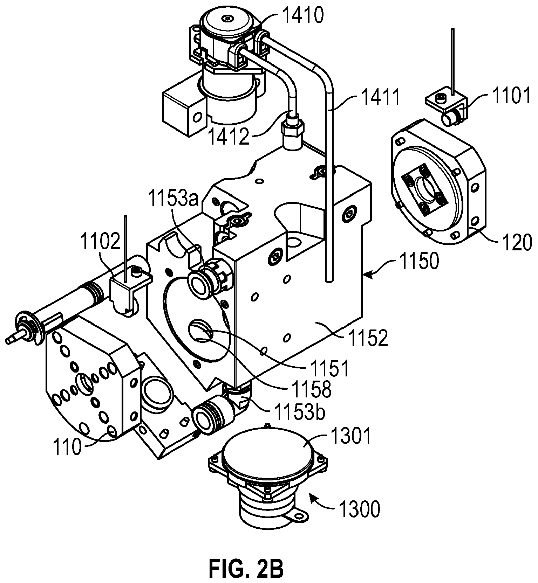

According to some embodiments, the sampling module **1150** may further include inlets and outlets (herein "sampling inlets/outlets") for enabling water sample replacement in the cuvette **1151** for measuring different WF states and locations (wherein each location may be associated with a specific treatment state of the WF or with a specific manipulation of sampled water). For example, as shown in **Fig. 2B,** the sampling module **1150** may include a sampling inlet **1153a** for allowing water arriving and channeled from one of the piping inlets of the flow control unit **1200** to enter the cuvette **1151,** and a sampling outlet **1153b** for allowing water to drain out of the cuvette **1151,** through one of the piping outlets of the flow control unit **1200.**

According to some embodiments, the sampling module **1150** may include other pump(s), inlet(s) and outlet(s) for example for enabling degasification of the water sample and/or for enabling insertion of alkali material(s) from the alkali container **1420** into the cuvette **1151** through the pump **1410** e.g. via one tube **1411** connecting between the container **1420** and the pump **1410** and another tube **1412** connecting between the pump **1410** and the cuvette **1151.**

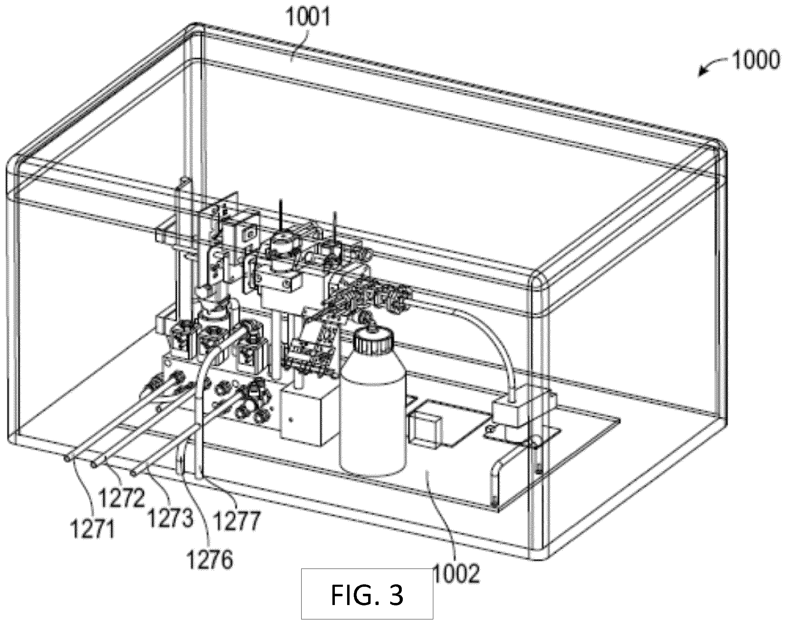

Fig. 3 shows a packaging unit **1001** for encasing and optionally also water-proofing parts of the apparatus **1000,** leaving only portions of the inlets and outlets such as inlets and/or outlets **1271-1275** extending outwardly from the packaging unit **1001** for channeling the water therethrough. The packaging unit **1001** may connect to the apparatus **1000** via a connecting plate **1002.\**

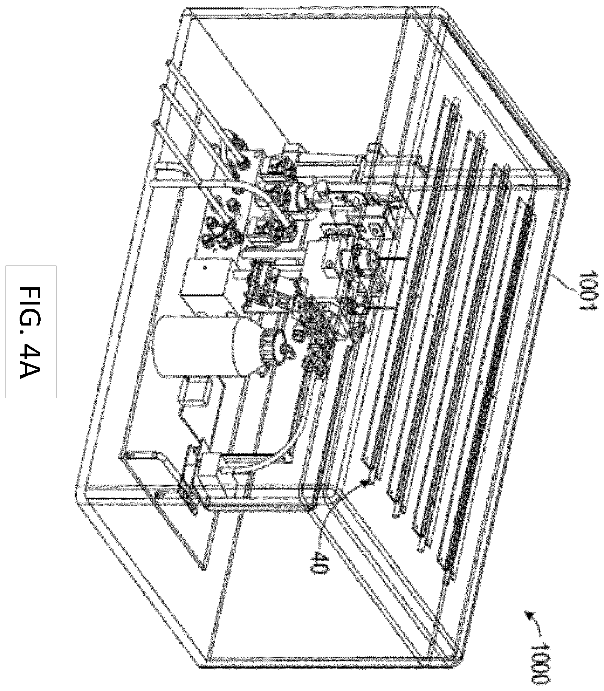

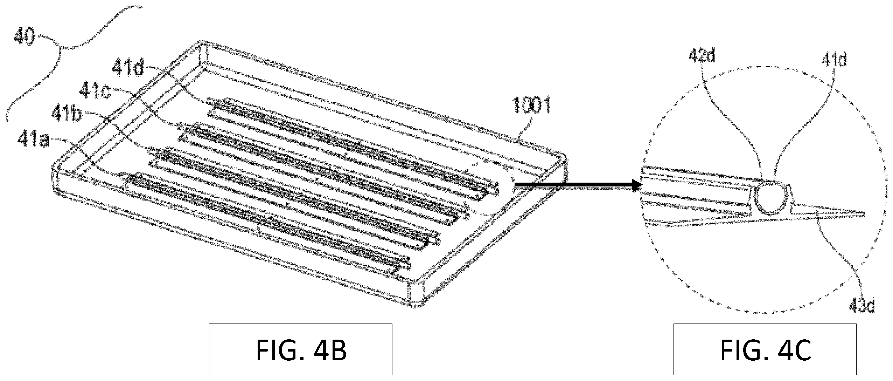

Fig. 4A shows a packaging unit **1001** having a cooling mechanism on an upper side thereof;

Figures 4A-4C show a cooling mechanism **40** installed/mounted over the packaging unit **1001** of the apparatus **1000,** according to some embodiments, for cooling the apparatus's devices and instruments such as the sensors, the PCB(s) etc. The cooling mechanism **40** may include multiple cooling channels such as cooling channels **41a, 41b, 41c** and **41d** each having a base structure such as base structure **43d** connected to a side of the packaging unit **1001** and holding and connecting to a cooling liquid or gas channeling tube such as tube **42d** together forming the cooling channel **41d** (see **Fig. 4C).**

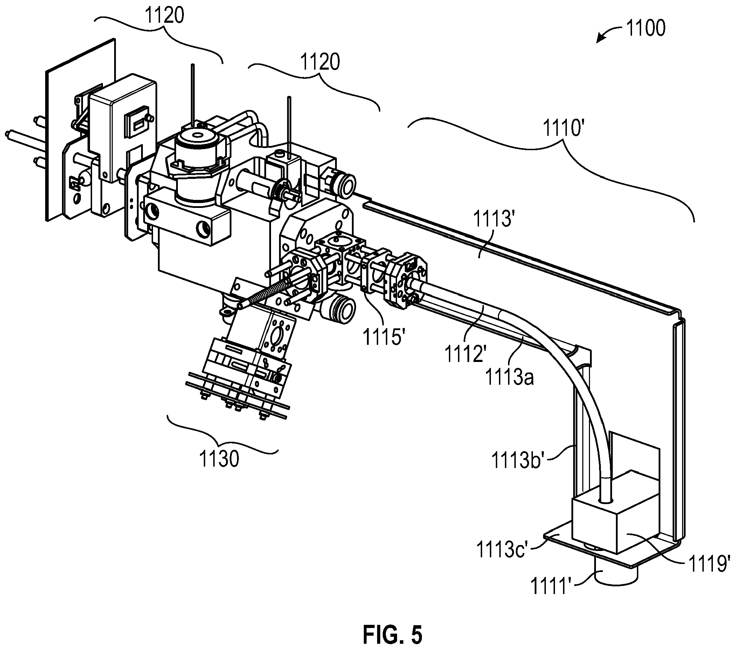

Fig. 5 shows a first optical module **1110'** having a non-straight fixture **1113'** locating its spectrometry device **1111'** angularly (e.g., perpendicularly) to the axis **x,** which is the optical axis of the output light exiting the sampling module **1150.** In this configuration, a flexible fiber guide cable **1112'** is used to guide an optical fiber therein, e.g. for guiding light emanating from the cuvette **1151** propagating at a direction parallel to the axis **x** through the optical setup **1115',** towards the spectrometry device **1111'** located angularly to the **x** axis by curving in respect to axis **x.**

According to some embodiments, the fixture **1113'** may be a curved or angular structure having one or more bracketing edges such as bracketing edges **1113a'** and **1113b'** for supporting one or more parts of the fiber guide cable **1112'.** The fixture **1113'** may also include a supporting plate **1113c'** for supporting the spectrometry device **1111'** and/or a processing unit **1119'** thereof.

According to some embodiments, water from the WF may be acquired and channeled into the cuvette **1151** at a specific water state by acquiring the WF water sample from a specific location of a WF piping (e.g. pre or post filtering by connecting and channeling water from a section of the WF piping that is before or after the filter of the WF). To measure a water sample of a different treatment type and/or treatment state (pre/post), the water sample of previous measurement may be first drained out of the cuvette **1151** before inserting a new sample by channeling water from the WF at a different location thereof.

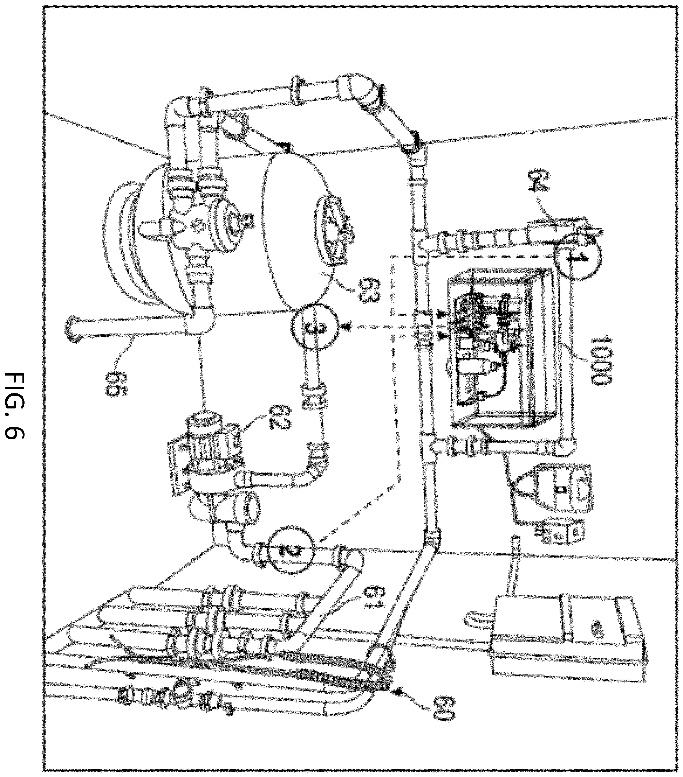

Reference is now made to **Fig. 6** schematically illustrating how the apparatus **1000** can be installed to a WF piping **60** for acquiring water samples from different locations in the WF via its WF piping **60** manifold by connecting to multiple different locations of the WF piping **60** and controlling flow from each location into the cuvette **1151** of the apparatus **1000** or and/or creating hydraulic connection to the pressure gauge **1170**

For example, as shown in **Fig. 6,** to acquire a water sample from a pre-pumping state (suction side) of the WF, one of the apparatus's **1000** inlets may connect (through a controllable valve) to a location **2** in the main pipeline **61** of the WF that is located before the WF's pump **62.**

Additionally or alternatively, as shown in **Fig. 6,** to acquire a water sample from a post-pumping and pre-filtering state of the WF water, one other inlet of the apparatus **1000** may connect (through a controllable valve) to a location **3** in the WF pipeline that is located after the WF's pump **62** and before the WF filter **63.**

Additionally or alternatively, to acquire a water sample from a post-chemical treatment (such as chlorination treatment), one other inlet of the apparatus **1000** may connect (through a controllable valve) to a location **1** in the WF pipeline through which a chemical substance (e.g. chlorine) is dispensed into the respective pipeline section via a dispenser **64.**

In the same manner, different inlets of the apparatus **1000** may connect to different sections of the WF pipeline **60** for acquiring water samples from the different WF piping **60** locations for measuring the various pre and/or post treatments states of the water. One or more outlets of the apparatus **1000** may connect to one or more drainage section(s) of the WF piping **60** such as section **65,** or to a separate drainage tube/channel of the apparatus **1000** itself.

According to some embodiments, the apparatus **1000** may be configured such as to allow easy installation and easy removal and replacing thereof by enabling easy positioning thereof and easy connecting thereof to the WF piping **60.**

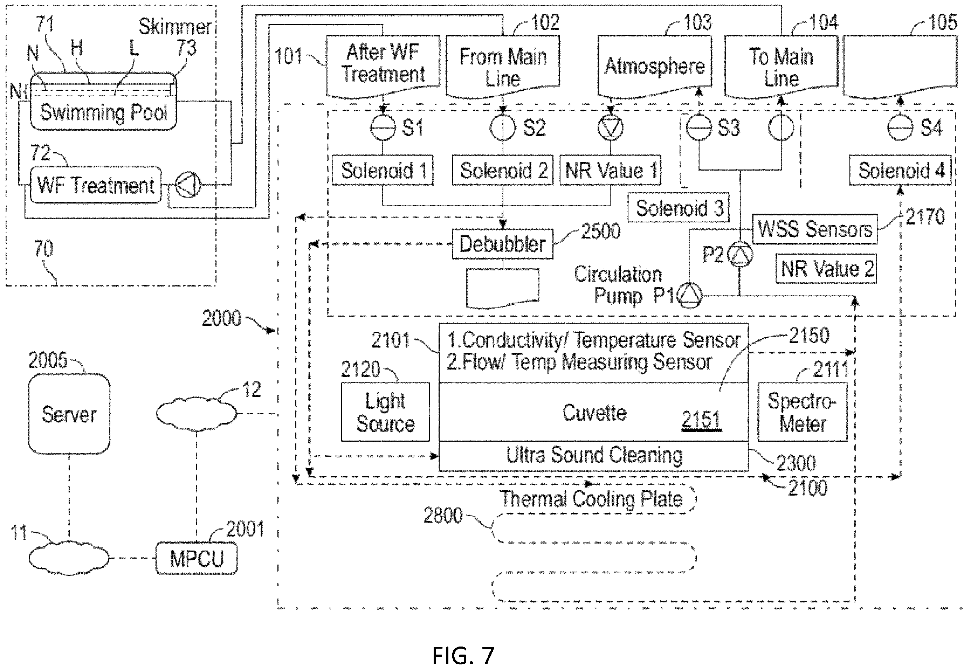

**Fig. 7** shows a schematic illustration of how an apparatus **2000** can connect to a WF **70,** where the apparatus **2000** may include:

(a) a detection unit **2100** including: (i) a sampling module **2150** having a cuvette **2151** for holding a water sample therein taken from water of a swimming pool **71** of the WF **70**; (ii) at least one optical module **2120** for at least emitting light towards the cuvette **2151**; (iii) a spectrometry device **2111** for measuring spectral characteristics of the water sample in the cuvette **2151**; (iv) and one or more sensors **2101** for measuring one or more other characteristics of the water sample, such as temperature, flow rate, turbidity, hardness, pollutants and/or disinfecting substances concentration/quantity, etc.;

(b) a cuvette cleaning mechanism **2300** such as ultrasonic (US) based cleaning treatment for cleaning the transparent windows and/or inner walls of the cuvette **2151** by applying US signals that enable detaching micro-organic contamination (such as algae) as well as inorganic contamination (such as calcium deposits) from the transparent windows and inner walls, using one or more US transducers;

(c) a degasification module **2500;**

(d) solenoid valves **S1-S4,** where:

S1 is located and configured to control flow from a location that is after WF treatment **72,** such as a pool filter or after a chemical dispenser (such as chlorine dispenser **101**) to the sampling module **2150;**

S2 is located and configured to control flow from a location before the pool filter in the main pipeline **102** to the sampling module **2150;**

S3 is located and configured to control flow from the sampling module **2150** to the main pipeline **104** or to a drainage line **103;** and

S4 is located and configured to control flow from the sampling module **2150** to a drainage pipe or outlet **105;**

(e) a cooling mechanism **2800** for cooling devices such as PCBs of the detection unit **2100;**

(f) one or more other sensors, pumps and valves such as WWS pressure sensor **2170** e.g., for measuring water flow rate/pressure in the piping area located after a pump **P1** and/or **P2** of the WF; and

(g) additional devices such as additional sensors and/or directing devices for acquiring measurements of parameters from the swimming pool WF **70** area such as skimmer **73** functionality state, water level of the water in the swimming pool reservoir (above, below or within normal level), main pump state etc.

The additional devices are often physically external to the apparatus 2000 and may include, for example, one or more of: pressure sensor(s), flow rate sensor(s), optical sensor(s), acoustic sensor(s), chemical sensors etc.

According to some embodiments, the electronically controllable solenoid valves **S1-S4** may be able to only be in two discrete states: fully open or fully closed, for example, or .in other embodiments, may be configured to enable non-discrete opening thereof to allow finer flow control. According to some embodiments, the apparatus **2000** may also include a MPCU **2001** in communication with all (or only some of) sensors of the detection unit **2100** such as the optical module(s) and the pressure sensor **2170,** for receiving sensor data from sensors of the detection unit **2100** and processing the received data to determine one or more characteristics of the WF **70.** The MPCU **2001** may have wire-based and/or wireless communication links to the sensors such as wire-based link **12.**

Additionally or alternatively, one or more remote servers such as cloud server **2005** may be in communication with the MPCU **2001** e.g. via a communication network **11,** for example, for using the MPCU to only locally control the data acquisition from the sensors and accumulate the acquired data and transmit the accumulated/acquired data in real time or on scheduled occasions to the remote cloud server **2005** for further processing and determination of at least some of the WF's characteristics or for display or further transmission of monitoring information indicative of condition of monitored parts of the WF.

For example, the MPCU **2001** may be used to process the received sensor data only to identify crucial acute problems in the WF **70** whereas the cloud server may be used for longer term storage of acquired data e.g., for identifying longer-term chronic problems and/or for improvement of algorithms based on machine-learning and for updating the algorithms in the MPCU **2001** based on accumulated knowledge from multiple water facilities etc.

According to some embodiments, as shown in **Fig. 7,** a first pipe connects through solenoid **S1** from main line receiving water after treatment (i.e. after filtration through pool filter and after chemical dosing e.g., acid dosage, salt chlorinator, and/or liquid chlorine dosage); a second pipe connects through solenoid **S2** from the main line, receiving water before treatment (i.e. after pump, before filtration through pool filter) ; a third pipe connects though solenoid **S3,** to the main line (under negative pressure due to main circulation pump suction during its operation); and a fourth pipe connects though solenoid **S4** to the atmosphere (drainage) for emptying the cuvette.

As shown, the pressure sensor **2170** is disposed and hydraulically connects the detection unit **2100** with the manifold of the pipes of the WF **70** via solenoids **S1-S4.**

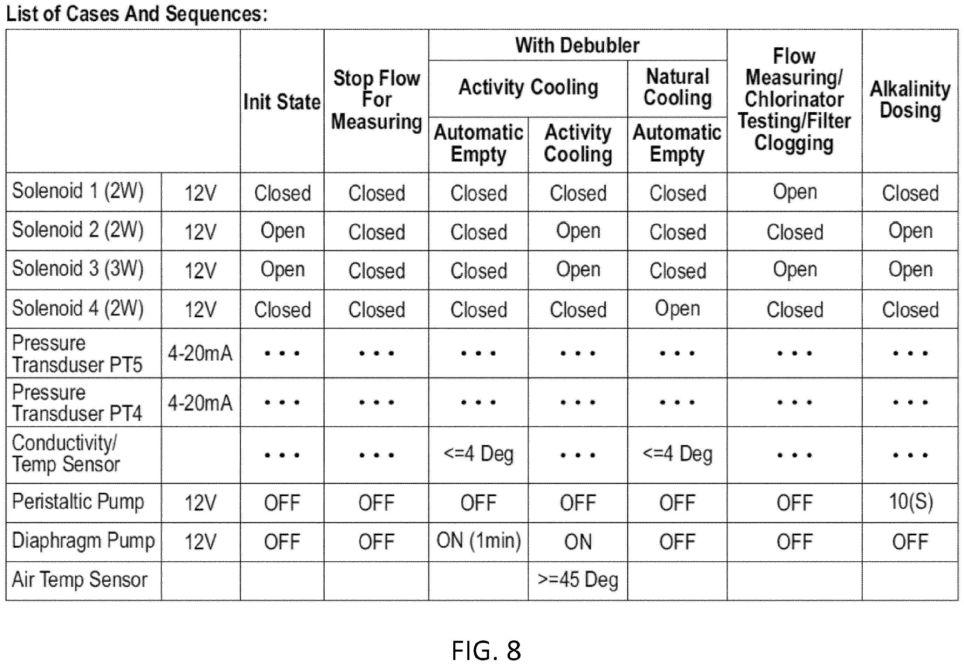

**Fig. 8** shows an exemplary list of solenoid valves' **S1-S4** states-combination (VSC) for various treatments and/or measurements acquisition purposes, for the system outlet shown in **Fig. 7,** according to some embodiments, for example, to test chlorinator filter state, the valves' states combination is: **S1** and **S3** are open and **S2** and **S4** are closed.

According to some embodiments, based on pressure sensing and optionally using also other sensing devices and techniques, such as the optical and/or ultrasonic based sensing, the apparatus **2000** may be configured to measure various characteristics of different locations, states, manipulations and/or states of the WF **70** such as:

[1] water level;
[2] filter state;
[3] backwash detection;
[4] flow in main line;
[5] skimmer state;
[6] chlorinator state;
[7] pump state.

The apparatus **2000** may perform measurements, using the first and second solenoids **S1-S2,** to control the water inlet and preforming time base sampling taking advantage of switching the sampling location before and after treatment.

## Water Level Measurement

According to some embodiments, the water level measurement may include (see Figure **10A):**
In skimmer-based pools, the water level should optimally be between 20-80 percent of the skimmer's level such that the pool water will always pass through the skimmer. The water level in respect to a known skimmer position and level may be measured before the pump (e.g., location **2,** Fig. **6,** position of before the pump and after the skimmer).

The water level measuring process may include, for example, the steps of:

(1.1) setting up the water level measurements by:

(a) calculating initial pressure $P_0$ at location **2,** when the WF pump is off, and there is no dynamic pressure due to flow of water due to the WF pump, where $P_0$ represents the pressure value at initial setup measurement;
(b) measuring/determining/receiving height of the skimmer (skimmer height **Sh** e.g., in cm) e.g., manually;
(c) determining initial water level $WL_0$ as a percentage of the skimmer's height (e.g., manually from observation) ;
(d) determining a reference water level $RWL_0$ (in cm), where $RWL = WL_0 \times Sh$ (for example, for a skimmer having a height of 10cm, and water level at zero flow rate initially measured as being at half the skimmer's height the reference water level will be:

$$RWL_0 = 10 \times 0.5 = 5cm$$

(e) equate the relating initial pressure measurements, $P_0$ with the current water level for the specific swimming pool (in relation to the skimmer height **Sh).**

(1.2) measuring current pressure P(t) at a future time at zero flow rate, from the same location **2** using the same pressure sensor;
(1.3) Calculate pressure difference (in millibar) $\Delta P(t) = |P(t) - P_0|$;
(1.4) Calculate the current water level or relative water level RWL(t) based on pressure difference $\Delta P(t)$;

The water level may be calculated in cm as follows:

$$RWLcm(t) = RWL_0 + \Delta P(t) * 0.9807 \text{ millibar/cm}$$

The water level may be calculated as a percentage of the skimmer's width as follows:

$$RWLpct(t) = RWLcm(t) / Sh$$

According to some embodiments, an alerting situation may be detected once any of the above calculated or measured parameter values exceeds a predefined threshold value and/or range of values. For example, an overflow of water caused by over-filling of the WF or insufficient amount of water in the WF caused by water evaporation or over drainage/pumping, may be detected if the water level determined is above a max value of the RWL or below a min value of the RWL. According to some embodiments the system may be set to detect associated $\Delta P(t)$ value exceeding predefined pressure threshold values $\Delta Pmin$ and $\Delta Pmax$, corresponding to water level maximum and minimum thresholds.

According to some embodiments once an overflow or an over-drainage situation is detected, the system may be configured to output alerts and/or automatically address the problem to mitigate it (e.g. by respectively draining or adding water to the WF.

According to some embodiments, the normal range of the water level in percentages, for a skimmer swimming pool WF, is typically between 20-80 percent (i.e. 20% < RWLpct(t) < 80%).

According to some embodiments, once an over filling or under filling of the WF is detected, an associated alerting visual indication may be outputted where each state over/under filled i.e. water level too low or too high - may be represented by a different indication color or using some other visual indication like a suitable graohic icon.

According to some embodiments, once a water-level problem is detected (too high or too low):

(i) an automatic recommendation may be displayed and/or transmitted to users such as the WF attending personnel; and/or
(ii) at least one automatic operation may be initiated and controllably operated by the system such as automatic filling/drainage of the WF container.

According to some embodiments, each automated controllable operation enabled by the system may be associated with one or more optional WF alerting states such that the same operation may be used to solve more than one WF impairments/situations and vice versa - a single detected problem may require several mitigating automated operations.

For example, automatic water drainage or filling operation can be done, for solving too high or too low skimmer water level.

In some cases water level issue (exceeding thresholds) may be associated with impairments in the WF devices or piping. For example low water level may be caused due to leakage in the piping or the pool itself. The system may also be configured to automatically check WF piping and/or devices such as filter, pump etc., when such alerting states are detected.

**Filter State Measurement**

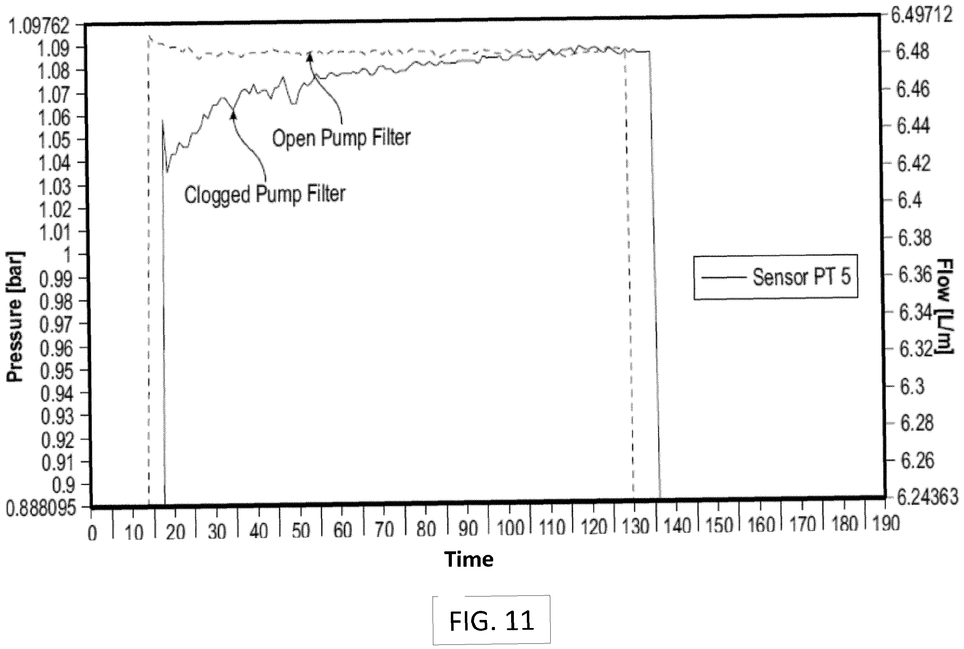

In swimming pools WFs that incorporate sand filters, backwash should be done regularly to keep the filter clean and prevent it from blocking. The below process, describes a method for detection of a filter state and alerting user(s) of the detected alerting state (Figures **10B, 10C** and **11),** according to some embodiments:
At setup 1:

(a) Denoting by $\Delta Pf_0$ a drop in pressure sensed in the filter, obtainable e.g., by detection of a pressure drop between two different pressure sensors located at different locations or alternatively via a single pressure sensor sensing at location **1** and location **3** via valves' control, to calculate the differential pressure across the pool's filter right after backwash (when the filter is clean), at time 0. This value may be calculated as:

$$\Delta Pf_0 = Pb_0 - Pa_0$$

This should be taken at a known water-flow rate and can be acquired at several times for achieving an averaged pressure difference.

$Pb_0$ (pressure before filter, after backwash, when filter is clean)
$Pa_0$ (pressure after filter, after backwash, when filter is clean)
At run time:
(b) periodically calculating the pressure drop on the filter: $\Delta Pf(t) = Pb(t) - Pa(t)$.

Pb(t) (pressure before filter, at time t of measurement)
a(t) (pressure after filter, at time t of measurement)
(c) Defining filter state (in percentages such that 100% represent a healthy full functioning filter) as:

$$FS = 100\% * (1 - [Pf(t) - Pf0] / PD) \text{ \{where PD is the pressure difference}$$

$$\text{between a non-clogged and fully clogged filter\}}$$

FS ≥ Threshold1 (such as 20%) ==> Blue (normal state)
Threshold2 (such as 5%) ≤ FS ≤ Threshold1 (such as 20%) ==> Orange (abnormal state - a back wash is needed)
FS < Threshold2 (such as 5%) ==> Black (hazardous state e.g., requiring shut sown of filter)

If value of pressure difference ΔPf(t) is too high (exceeds a maximum threshold), a gradual build-up of pressure in the pool's filter (e.g. sand filter, cartridge filter) may indicate that the filter is becoming occluded. This trend may coincide with increasing water pump electric current consumption and with an increase in water turbidity. This condition requires maintenance actions, such as performing a filter backwash. Therefore an increase of pressure values' difference between measurements before and after the filter locations may be used for detection of filter states (clogged, fully open, and/or partially open), and alert users such as pool maintainers regarding the need for filter cleaning.

Other conditions can also be used to monitor filter health such as when the FS becomes greater than 100%, this may indicate the pressure has dropped and that "channelling" may have occurred in the filter - a state of poor filtration that may require replacing the filter media.

## Backwash Detection

According to some embodiments, a backwash state/rate of the filter may be detected in real time and backwash rate/state can be used to estimate several useful WF properties.

## Typical Backwash Procedure

(i) Pump is turned off.
(ii) Faucet is changed to "Backwash".
(iii) Pump is turned on for the duration of the backwash (nominally, this stage may take between 30 sec to 2 min, although this can vary depending on other factors).
(iv) Pump is turned off.
(v) Faucet is changed to "Rinse".
(vi) Pump is turned on (nominally this stage can take about half of the entire procedure time).
(vii) Pump is turned off.
(viii) Faucet is changed to "Filtration" (nominal operation)

A backwash procedure is detected for all times in which the following conditions are met:

(1) Flow = 0 (or below a very low threshold, in case some pumps exhibit noise even when turned off)
(2) Pb(t) > 0.25 * MA_Pb (moving average of Pb during recent flow)

These conditions together guarantee that the pump is in an operational state (turned on) - otherwise Pb(t) should be around zero, while at the same time no flow is detected (as the sensor is located after the filter and the water flow is directed to the drainage).

Additional conditions may be added that should only hold during backwash procedure, such as:

(a) Pa(t) should remain around 0 (and in any case significantly below its nominal value during normal operation) for the duration of the procedure as long as the faucet is in "Backwash" or in "Rinse". This condition can and should replace the first condition for those swimming pools WFs, in which the flow sensor is located before the filter.
(b) Pb(t) should be around 0 just before the actual backwash and rinse (pump off and change in the state of the faucet). There should be two separate "hills" or peaks in Pb(t) readings (with regards to its ~0 surroundings). The first corresponds to the backwash state and the other corresponds to the rinse. Both are counted as "backwash", since for our purposes they are very similar, as water flows into the drainage.

*Therefore, if:*

*Pb(t) - Pb(t-1) < -2\*sigma (in Pb measurements) AND Pa(t) - Pa(t-1) < - 2 \*sigma (in Pa measurements)*
*Then a start point of a backwash is detected at time t.*

According to some embodiments, the pressure measurements should be averaged over short periods.
According to some embodiments, backwash measurements can enable estimating water loss, for example, by:

(i) determining the duration of the backwash based on detecting the start and end points of the backwash; calculating the integral of backwash flow rate(t) over the time interval: startbackwash-endbackwash which amounts to the backwash water volume (volume loss)

Optionally also, estimating the surface area of the swimming pool WF via:

(a) calculation of the volume loss as described above;
(b) calculation of the change in water level when the pump is off, right after the backwash is done (according to Water Level Measurement procedure)

Pool Surface Area ($m^2$) = Backwash Water Volume ($m^3$) / Drop in Water Level (m) from before to after the backwash
According to some embodiments, measurements data may be continuously or occasionally transmitted to a cloud server or any other remote computing and communication device/system, for further analysis, storage, display and the like. For example using machine learning algorithms for deducing the WF characteristics and for automatic operation of WF maintenance and/or transmission of information indicative of alarming situations and/or the WF state to authorized WF users/managers, caretakers etc. via their personal end devices.
According to some embodiments, data of the pressure measurements may be correlated with additional measurements such as water pump current/power consumption, to evaluate the efficiency of the pump and the need for a backwash.
According to some embodiments, sensor data from the pressure sensor and optionally also optical sensors data may be correlated with external data sources such as weather forecasts, to assess maintenance actions such as the need for water addition, chlorine dispensing quantities etc.
Pressure data can undergo trends and changes analysis, to ascertain whether immediate action should be taken.
According to some embodiments, the cloud server **2005** may be configured to:

produce requirements/actions/operation plans for maintenance of the WF **70** e.g., in the
rm of automatic operation control, alerts to the responsible people and the like;
update the MPCU **2001** analysis programs/algorithms based on accumulated information and machine learning based analysis, so that the MPCU **2001** is able to optimize identification and reaction to emergency scenarios (e.g. identify a ruptured pipe condition, and shut specific valves to avoid water loss); and/or

**Alkalinity Measurement**

The apparatus **2000** and system including the apparatus **2000** and the cloud sever **2005** may be further configured to:use a micro pump for dispensing acidic and/or alkali titrants (such as NaOH as a base) to sampled pool water, thus implementing a titration process by pumping from a titrant bottle into a sampling water container (e.g., cuvette **2151**) of the sampling module **2150**;

mixing (e.g., via pumping) the solution of the water sample and the added titrant in the cuvette;
using spectral and/or chemometrics algorithms and/or other pH measurement for measuring changes in the pH of the mixed solution, e.g., to determine the pool water's Alkalinity Buffer Intensity, $\beta = d[OH]/dpH$.

Optionally, the systems, apparatuses and methods may also be using a Halochromic indicator, brought in direct contact with the examined solution in the sampling module **2150,** and indicating the pH level by a change in its color, optical absorbance or fluorescence properties, using optical detector(s) such as a spectrometer of the detection unit **2100,** by illuminating the sample (solution) in the partially or fully transparent cuvette **2151.**The calculated/measured Alkalinity/pH information may be propagated to the cloud server **2005,** which may be configured to use one or more machine learning algorithms in order to determine an optimal WF maintenance plan. This maintenance plan may be able to help ensure that: (i) the pH, LSI, concentrations of Chlorine compounds and Cyanuric acid are kept optimal; (ii) the water of the WF (e.g., swimming pool) is optimally consumed or used, avoiding unnecessary water waste.

**Leak Detection**

According to some embodiments, the system/apparatus may also be configured to detect leakages, using a Leak Detection Algorithm configured to:

(i) Track water level changes over time;
Exclude water level changes due to backwash volume (detected);
Exclude water level changes due to water evaporation (estimated);
Evaporation Rate may be modeled as a function of: Water Temperature, Humidity, Wind Velocity, Pool Surface Area;
Monitor residual changes in water level: unaccounted for continuous (approximately linear) drops in water level over time indicate high probability of leaks

**Treatment (Acid Dosage, Liquid Dosage, Chlorinator) State**

According to some embodiments, the system/apparatus may also be configured to detect WF Treatment state. For example by measuring via a spectrometer and chemometrics. Free Chlorine levels in the water sampled from locations of the WF at a given time t, before, FCb(t) and after treatment FCa(t) during operation and periodically over long-term operation to determine, for example one or more of:

Chlorinator Hours of Operation
Level, ratio or gap of free chlorine level during Hours of Operation
$\Delta FC(t) > 0$, where $\Delta FC(t) = FCa(t) - FCb(t)$.
Chlorinator Production Output Level

a. The degree of $\Delta FC(t)$ being correlated to chlorinator production output level
b. Changes (in particular declines) in the chlorinator production output level may indicate the need for cleaning of electrodes, or for constriction of flow.

Alerts may be sent to one or more users to perform maintenance actions such as to refill the Acid or Chlorine buckets, fix problems in devices such as dosing connections, dosing pump, etc.

**Fig. 9** is a block diagram, showing valves and piping connections of a system for measuring characteristics of a water facility connecting to various piping areas of a piping of a water facility using multiple sensors such as pressure sensors for detection of WF functionality state e.g., for WF impairments detection, according to some embodiments;

**Figures 10A-10C** show outputs behavior of various pressure sensors, located and configured to measure the WF in various locations in different states of the measured WF location, according to some embodiments, using the system layout of **Fig. 9: Fig. 10A** shows exemplary output of sensor PT4 for measuring level of the water in a swimming pool WF; **Fig. 10B** shows exemplary outputs of sensors PT5 and PT7 for measuring clogging by measuring differences between two sensors' output, where the sensors are located at different strategic locations; **Fig. 10C** shows that the clogging of the pump filter is manifested in an enlarged difference between the sensors' outputs;

Some of the measurements may be carried out by using a single sensor located at a single location over the WF pipeline for example, to measure general state of the WF (regardless of treatment thereof) such as a single pressure sensor **PT4** to be able to identify normal state and impairments thereof. For example, as shown in **Fig. 10A** to determine water level state based on pressure sensed by **PT4.**

In other cases, two sensors such as pressure sensors **PT5,** located after the filter and **PT7,** located before the filter, may be used to assess filter operation by comparing the pressure output before and after being passed through the filter, e.g., to detect impairments such as clogging of the filter and also to detect severity of the impairment. **Figures 10B** and **10C** show outputs difference of about 0.2 bars in a state in which the filter is unclogged or has a minor clogging **(Fig. 10B)** and a difference of 0.7-0.8 bars, in a case of a severe clogging of the filter **(Fig. 10C)**.

**Fig. 11** shows a pressure behavior for a clogged and unclogged pump filter that can be used to detect clogging impairments in the WF by use of a single pressure sensor **PT5** placed after the pump filter, according to some embodiments. In this case a normal unclogged pattern of the pressure vs. time curve may be compared with monitored pressure sensor output to determine filter and/or pump state.

Aspects of disclosed embodiments pertain to methods for measuring various characteristics of water of a water facility (WF) including at least the steps of:

providing a flow control unit comprising at least: (i) multiple piping inlets and outlets for connecting thereby to different parts located at different locations of the WF piping setup; and (ii) multiple electronically controllable valves, each of the electronically controllable valves being deployed at a different inlet or outlet of the multiple piping inlets and outlets, such as to control water flow to and from different parts located at different locations of the WF piping setup; providing a detection unit comprising a sampling module and at least one optical module being configured and located to optically detect characteristics of the water of the WF; and controlling operation of the valves, for measuring one or more characteristics of a selected detection location of the WF piping setup, in a selected pre-treatment state or post-treatment state of the WF water, wherein at least some of the treatment states are each associated with a specific detection location.

According to some embodiments, the method may further include automatic and/or manually controllable selecting of the specific treatment and the specific post-treatment or pre-treatment state of the WF water prior to controlling the valves.

According to some embodiments, the controlling of the valves may be done electronically via a main control and processing unit (MCPU) associated with the detection unit and with the flow control unit by adjusting the state of each of the valves.

According to some embodiments one or more of the light sources used for optical detection of characteristics of the WF may be irradiating light within the ultraviolet (UV), infrared (IR) and/or visible light wavelength band.

According to some embodiments the method may further comprise water sample replacement done by channeling water sample no longer relevant for use out of the cuvette and channeling water sampled from the desired location in the WF into the cuvette of the apparatus **1000** or **2000,** for example.

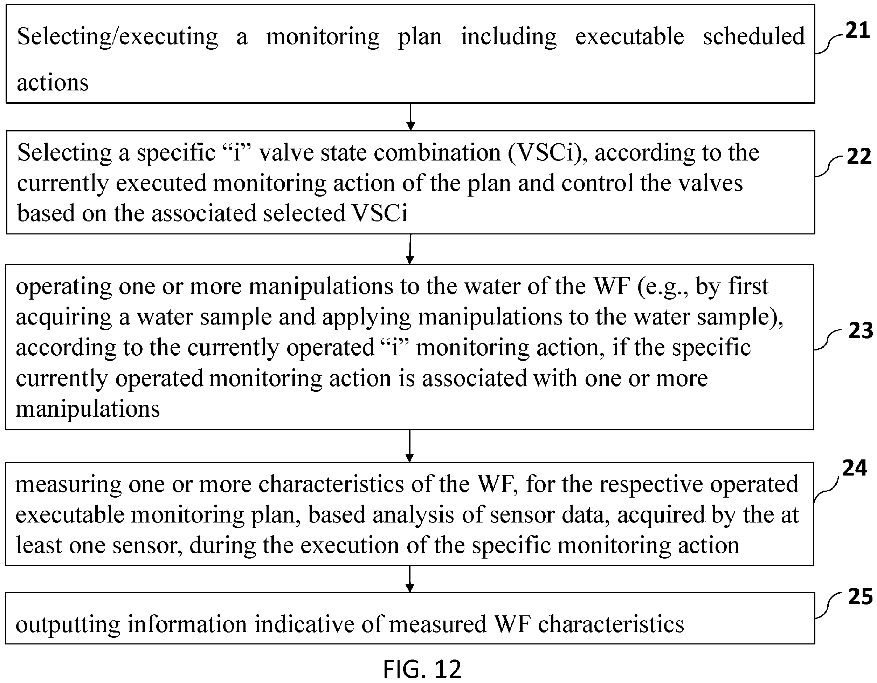

Reference is now made to **Fig. 12,** schematically illustrating general steps of a process for monitoring a WF by measuring WF characteristics using a detection unit that includes at least one pressure sensor and/or other detectors/sensors for optical and/or acoustic measuring of WF characteristic, according to some embodiments.

The process may include, for example, at least: selecting/executing a monitoring plan including executable scheduled actions (step **21);**

selecting a specific "i" valve state combination (VSCi), according to the currently executed monitoring action of the plan and control the valves based on the associated selected VSCi (step **22);**
operating one or more manipulations to the water of the WF (e.g., by first acquiring a water sample and applying manipulations to the water sample), according to the currently operated "i" monitoring action, if the specific currently operated monitoring action is associated with one or more manipulate (step **23);**
measuring one or more characteristics of the WF, for the respective operated executable monitoring plan, based analysis of sensor data, acquired by the at least one sensor, during the execution of the specific monitoring action (step **24);** and
outputting information indicative of measured WF characteristics (step **25).**

According to some embodiments, the method may further include generating recommendations (such as recommended actions that can be manually and/or automatically executed/performed) for how to maintain the WF based on detected WF characteristics and/or automatically executing operation commands (e.g. combined as a maintenance plan) for maintenance of the WF (such as adding water, drainage of water, automatic shutting down of devices of the WF that are broken or in danger of being damaged etc.).

According to some embodiments, some characteristics of the water sample such as opacity or turbidity level, concentration of substances etc. e.g., may be measured by using spectrometry.

According to some embodiments, the systems and apparatuses of the present invention may further be configured to conduct measurements requiring extended monitoring periods of each water sample e.g., in cases requiring substances such as biomass or crystalizing materials to be cultivated/grown/incubated e.g., for several days in order to determine various characteristics of the water such as type of biological and/or chemical pollutants, testing for optimal quantities and dosing of additives for water purification, testing for various conditions for biological (organic and natural) water filtration and cleansing etc. For example, a water sample may be delivered/directed to a second sampling module of the system/apparatus (not shown) for algae incubation of several hours or days and optionally for testing algae growth retardants or extermination processes, in order to determine the types of algae growing in the water and how its growth can be avoided or delayed in the most optimal and cost and time-effective manner.

Another example may be to have a water sample undergo a passive or active dechlorination via one or more of: natural chlorine evaporation, controlled heating, spraying, bubbling, specialty filtering and the like, e.g., for determining one or more characteristics of the water as they would have been without the chlorine addition.

Yet another example is to alter the pH of the water sample, to deliberately change the ratios between pH dependent species in the sample and thus assist in detecting them via spectroscopic means.

All or some monitoring and maintenance operations of the system/apparatus and parts thereof may be automatically controllable and done for enabling unmanned automatic WF maintenance, impairments and problems detections and alerting and optionally also at least partial automatic repair of detected impairments such as automatic flow control (increase or decrease) for clogging repair or mitigation, automatic addition of sterilization materials such as chlorine, automatic adding of water to the WF for regulating water level.

According to some embodiments, the apparatus of any one of the embodiments shown above such as apparatus **1000** may further include one or more additional sampling modules such as one or more additional cuvettes and/or one or more additional water chambers, for enabling conducting testing of water samples in various conditions and/or under various manipulations done to the water sample(s) such as for enabling long incubation of biomass in the sample for testing various characteristics of the biomass such as biomass growth rate, biomass growth-inducing vs. growth-diminishing conditions and the like. The water sample in the original sample module or the additional sample module may be manipulated for testing/measuring the various characteristics of water contained thereby, using one or more additional manipulation tools such as a heater, a cooler, a substances dispenser, etc. The apparatus may further include one or more separate optical and/or other devices, for measuring the outcomes of the water sample manipulations done using the additional sample modules.

In some embodiments, the additional sampling module(s) may be used for applying one or more manipulations over the water sample therein, such as biomass incubation, temperature changes applied over the sample, mixing of the water sample with one or more substances, etc., where the actual measurements of the characteristic of the water sample after (post) its manipulation(s) ("manipulated sample") may be carried out by the one or more optical modules of the apparatus such as via the spectrometer, by channeling the manipulated water sample back to the first sampling module (e.g. cuvette).

The entire process of channeling of the water sample(s) towards the additional/first sampling module(s) applying the manipulation(s) over the sample(s) and measuring post-manipulation characteristics of the manipulated sample(s) can be carried out automatically, semi automatically and/or computer-controllable e.g., by a software configured for automotive operation and testing and/or by user(s) via a designated user interface.

EXAMPLES

Example 1 is an apparatus for measuring characteristics of a water facility (WF), the WF comprising at least a piping setup, the apparatus comprising:

> |a flow control unit comprising at least:
> multiple piping inlets and outlets connecting to different parts of the WF; and
> multiple controllable valves;
> a detection unit comprising at least one sensor; and
> a main processing and control unit (MPCU), operatively associated with the flow control unit and with the detection unit,
> wherein the MPCU is configured to receive and analyze sensor data from the at least one sensor of the detection unit and to control operation of the controllable valves, for applying an action by selection of different valves states combinations (VSCs), each VSC being associated with a different location and/or a different state of the WF and/or water thereof.

In example 2, the subject matter of example 1 may include, wherein the at least one sensor is selected from: pressure sensor, optical sensor, spectroscopic sensor, spectrometer.

In example 3, the subject matter of any one or more of examples 1 to 2 may include, wherein the detection unit further comprises: (i) a sampling module having a water sample holder that is configured for filling and drainage of the sample holder with water from different locations of the WF using the valves control; and (ii) at least one sensor for sensing characteristics of the water sample.

In example 4, the subject matter of example 3 may include, wherein the detection unit further comprises at least one optical module configured for illuminating the water sample in the sample holder and one or more optical sensors for optically sensing characteristics of the illuminated water sample.

In example 5, the subject matter of example 4 may include, wherein the detection unit further comprises additional sensors for sensing one or more of: temperature of the water sample, turbidity level of the water sample; hardness of the water sample, concentration and/or quantity of substances.

In example 6, the subject matter of any one or more of examples 4 to 5 may include, wherein the sample holder comprises a partially or fully transparent cuvette for holding therein water sampled from the WF, and wherein the at least one optical module comprises at least:

a first optical module configured to measure spectral characteristics of light emanating from the water sample;
a second optical module having one or more light sources for illuminating the water sample in the cuvette; and/or
a third optical module having one or more light sources for illuminating the water sample in the cuvette.

In example 7, the subject matter of example 6 may include, wherein the sampling module further comprises a non-transparent casing having multiple windows for directing light emanated from one or more light sources of the at least one optical module, towards the cuvette and/or towards one or more optical detectors of the at least one optical module.

In example 8, the subject matter of any one or more of examples 4 to 7 may include, wherein the at least one optical module comprises:

at least one light source for irradiating the water sample; and
at least one optical detector, configured to optically detect characteristics of the water sample in the sampling module, by detection of characteristics of output light outputted from the water sample.

In example 9, the subject matter of example 8 may include, wherein the at least one optical detector comprises at least one of: a spectral optical detector, a photodetector, a photodiode, a charged coupled device (CCD) camera, an RGB sensor.

In example 10, the subject matter of any one or more of examples 7 to 9 may include, wherein the first optical module comprises a spectrometry device and a processor for spectral analysis of light detected by the spectrometry device.

In example 11, the subject matter of example 10 may include, wherein each of the processor of the second optical module and/or of the third optical module comprises a printed circuit board (PCB), configured to controllably operate each light source.

In example 12, the subject matter of any one or more of examples 6 to 11 may include, wherein each light source of each of the second and/or third optical module is configured to irradiate light in any one or more of: the ultraviolet (UV) and/or the visible (VIS) spectral band.

In example 13, the subject matter of any one or more of examples 6 to 12 may include, wherein at least one of the second and third optical modules comprises one or more optical detectors and one or more optical elements for directing light emanating from the one or more light sources towards the one or more optical detectors of the respective second or third optical modules.

In example 14, the subject matter of any one or more of examples 1 to 13 may include, wherein the different WF states or locations comprise one or more of:

pre and/or post filtering treatment of the WF water;
pre and/or post backwash treatment of the filter of the WF;
pre and/or post degassing of the WF water;
pre and/or post cooling or heating of the WF water;
pre and/or post drainage of the WF water.

In example 15, the subject matter of any one or more of examples 1 to 14 may include, wherein the one or more manipulation applied to the water of the WF, comprise one or more of:

one or more pre and/or post chemical treatment of at least one water sample obtained from the WF;
one or more pre and/or post biomass incubation in at least one water sample obtained from the WF;
one or more pre and/or post applying of acoustic signals to at least one water sample obtained from the WF.

In example 16, the subject matter of any one or more of examples 1 to 15 may include, wherein the apparatus further comprises a degasification module.

In example 17, the subject matter of any one or more of examples 1 to 16 may include, wherein the apparatus further comprises one or more acoustic transducers for applying an acoustic signal to water in a sampling module of the apparatus having a water sample holder for holding therein a sample of the WF water, the one or more acoustic transducers being used for detection of one or more characteristics of the WF and/or for cleaning of the interior of sample holder.

In example 18, the subject matter of example 17 may include, wherein at least one of the one or more acoustic transducers is configured for outputting and/or detection of ultrasonic (US) acoustic signals.

In example 19, the subject matter of example 18 may include, wherein the at least one US transducer is used for one or more of: inhibition of algae/microalgae growth in the sampling module and/or in one or more other places in the apparatus and/or in the WF, detection of water hardness level, detection of flow, estimation of water temperature.

In example 20, the subject matter of any one or more of examples 1 to 19 may include, wherein the one or more characteristics of the WF comprising one or more of: water hardness, concentration and/or quantity of organic and/or chemical substances, WF devices' functionality, WF piping condition.

In example 21, the subject matter of any one or more of examples 1 to 20 may include, further comprising one or more acoustic transducers and/or acoustic detectors for acoustically detecting one or more characteristics of the WF including at least acoustic detection of functioning of devices of the WF including at least functioning of the WF's filter and/or pump.

In example 22, the subject matter of any one or more of examples 1 to 21 may include, wherein the apparatus further comprises at least one thermal detector for measuring temperature of water of the WF and/or for measuring temperature of a water sample held by the sampling module and/or any other part of the WF.

In example 23, the subject matter of any one or more of examples 1 to 22 may include, wherein the apparatus further comprises an alkalinity module for controlling and/or measuring pH level of the water in the WF, and/or converting HOCl to OCl for the purpose of measuring same, and/or for estimating background water spectrum.

In example 24, the subject matter of example 23 may include, wherein the alkalinity module is associated with the sampling module and includes a container for containing therein alkali and/or acidic material and at least one means for dispensing the material from the container into a sampling module of the apparatus, the sampling module being configured to acquire water samples from the WF.

In example 25, the subject matter of any one or more of examples 1 to 24 may include, wherein the apparatus further comprises a packaging unit for encasing therein all or part of other modules and units of the apparatus.

In example 26, the subject matter of any one or more of examples 1 to 25 may include, wherein the apparatus further comprises a cooling mechanism comprising one or more cooling channels enabling channeling of a cooling liquid or gas for cooling parts of the apparatus.

In example 27, the subject matter of example 26 may include, wherein the cooling mechanism is embedded in or attached to a packaging unit of the apparatus, the packaging unit being configured to encase all modules and other units of the apparatus.

In example 28, the subject matter of any one or more of examples 1 to 27 may include, wherein the MPCU is further configured to transmit information indicative of measured WF characteristics to one or more remote devices and/or systems via one or more communication links, for enabling the transmitted information to be presented, displayed, printed, indicated, further processed and/or stored by the external devices and/or systems.

In example 29, the subject matter of example 28 may include, wherein at least some of the valves of the flow control unit comprise electronically controllable solenoid valves.

In example 30, the subject matter of any one or more of examples 1 to 29 may include, wherein the MPCU is further configured to:

operate an executable monitoring plan including executable scheduled monitoring actions;
select detection location "Li" for each monitoring action and an associated valves states combination I (VSCi);
control direction and/or interconnection of water from the selected detection location Li to one or more sensors or a sampling module of the apparatus by controlling the valves according to the selected VSCi;
for each of at least some of WF parts' characteristics: acquire sensor data from the at least one sensor of the apparatus for different locations and/or state of the WF according to the executable monitoring plan;
analyze the acquired sensor data to detect one or more characteristics of the WF, based on the sensor data, using one or more analysis algorithms; and
output information indicative of detected WF characteristics.

In example 31, the subject matter of example 30 may include, wherein the MPCU is further configured to one or more of: transmitting, displaying, further processing and/or storing the outputted information.

In example 32, the subject matter of example 31 may include, wherein at least one of the one or more algorithms used for sensor data analysis comprises a machine learning algorithm and/or an algorithm derived from machine learning.

In example 33, the subject matter of any one or more of examples 1 to 32 may include, wherein the apparatus further comprises an installation mechanism for removable installation of the apparatus to the WF piping.

In example 34, the subject matter of any one or more of examples 1 to 33 may include, wherein the WF comprises at least one skimmer, and wherein the at least one pressure sensor of the apparatus is further used for detection of updated skimmer water level.

In example 35, the subject matter of example 34 may include, wherein the updated skimmer water level is detectable by using at least one known or premeasured reference pressure value P0 associated with a premeasured skimmer water level of the skimmer of the specific WF.

In example 36, the subject matter of any one or more of examples 1 to 35 may include, wherein the WF comprises at least one filter and at least one pump, wherein the apparatus is further configured to determine state of the at least one filter and/or the at least one pump based on received sensor data from at least one pressure sensor.

In example 37, the subject matter of example 36 may include, wherein the filter state is detectable by using at least two pressure sensors, one located before the filter and the other located after the filter.

In example 38, the subject matter of example 37 may include, wherein pressure measurements for determining filter state are taken in one or more of the following filter sides and/or operation states: before the filter, after the filter, after backwash.

In example 39, the subject matter of any one or more of examples 1 to 38 may include, wherein the WF characteristics are further measured by measuring alkalinity of a water sample taken from the WF, the alkalinity measurement being conducted based on pH and/or spectral measurements of a mixed solution comprising a mixture of a sample of the water from the WF and an added titrant.

In example 40, the subject matter of any one or more of examples 1 to 39 may include, wherein the apparatus is further configured for detection of leakages in the WF, using a leak detection algorithm, based on detection of changes in water level over time and exclusion of other causes for water level changes, selected from the exclusion causes of: backwash volume changes, water evaporation.

In example 41, the subject matter of any one or more of examples 1 to 40 may include, wherein the apparatus is further configured to apply one or more manipulations over one or more water samples taken from the WF and measure the one or more water samples after being manipulated.

In example 42, the subject matter of example 41 may include, wherein the one or more manipulations is selected from the following list of:

incubating the water sample for a selected incubation time for testing biomass growth related characteristics;
adding one or more substances to the water sample;
changing temperature of the water sample;
removing one or more substances (such as Chlorine) from the water sample;
changing pH of the water sample.

In example 43, the subject matter of any one or more of examples 41 to 42 may include, wherein the apparatus further comprises one or more additional sampling modules for holding each therein an additional water sample and performing manipulation thereupon; and/or one or more additional measuring devices for measuring one or more characteristics of one or more manipulated water samples.\

Example 44 is a method for measuring various characteristics of water of a water facility (WF), the WF comprising at least a piping setup, the method comprising at least:

provide at least one sensor configured to detect one or more characteristics of the WF;
operate an executable monitoring plan including executable scheduled monitoring actions, wherein each monitoring action is associated with a specific valves states combination and with a specific location in the WF and/or with one or more specific manipulations applicable to water from the WF;
select a specific "i" valve state combination (VSCi), according to the currently executed monitoring action and control the valves based on the selected VSCi associated with the specific currently executed monitoring action;
operate one or more manipulations to the water of the WF, according to the currently operated monitoring action, if the specific currently operated monitoring action is associated with one or more manipulations;
measure one or more characteristics of the WF, for the respective operated executable monitoring plan, based on analysis of sensor data, acquired by the at least one sensor, during the execution of the specific monitoring action; and
output information indicative of measured WF characteristics.

Example 45 is a system for measuring characteristics of a water facility (WF), the WF comprising at least a piping setup, the system comprising:

(i) an apparatus comprising:

- a flow control unit comprising at least:

multiple piping inlets and outlets connecting to different parts of the WF; and
multiple controllable valves, each of the controllable valves being deployed at a different inlet or outlet of the multiple inlets and outlets, such as to control water flow and/or interconnection to and from different parts of the WF, located at different locations of the WF piping setup;

- a detection unit comprising at least one sensor; and

- a main processing and control unit (MPCU), operatively associated with the flow control unit and with the detection unit configured to receive and/or generate information indicative of one or more WF characteristics based on acquired sensor data from the at least one sensor, acquired for different locations and/or states of the WF, wherein the MPCU and the flow control unit are configured to control operation of the controllable valves, for measuring different locations and/or states of the WF by selection of different valves states combinations (VSCs), each VSC being associated with a different location and/or a different state of the WF and/or a different manipulation being performed in the apparatus; and

(ii) at least one remote device configured to receive the information generated by the MPCU and use the received generated information for one or more of: displaying, further analyzing, storing, printing and/or updating analysis algorithms of the PCU used for sensor data analysis.

Example 46 is a method for measuring characteristics of a water facility (WF) using one or more pressure sensors, the method comprising at least:

(a) acquiring pressure measurements from at least two different locations in the WF
(b) determining a state of at least one WF part, based on the acquired pressure measurements;
(c) determining one or more recommendations for maintaining the WF, based on determined state of each of the at least one WF part.

In example 47, the subject matter of example 46 may include, wherein the method further comprises determining operation commands for automatic maintaining of the at least one WF part, based on the determined maintenance recommendations.

In example 48, the subject matter of any one or more of examples 46 to 47 may include, wherein the method further comprises detecting water level of a skimmer of the WF, by using at least one pressure sensor measurements.

In example 49, the subject matter of example 48 may include, wherein the skimmer water level is detected by using at least one known or premeasured reference pressure value $P0$ associated with a measured skimmer water level of the specific skimmer of the specific WF.

In example 50, the subject matter of any one or more of examples 46 to 49 may include, wherein the method further comprises determining or detecting state of each filter and/or each pump of the WF, based on received sensor data from at least one pressure sensor.

In example 51, the subject matter of example 50 may include, wherein the filter state is detectable by using at least two pressure sensors, one located before the filter and the other located after the filter.

In example 52, the subject matter of example 51 may include, wherein pressure measurements for determining or detecting filter state are taken in one or more of the following filter sides and/or operation states: before the filter, after the filter, after backwash.

In example 53, the subject matter of any one or more of examples 46 to 52 may include, wherein the WF characteristics are further measured by measuring alkalinity of a water sample taken from the WF, the alkalinity measurement being conducted based on pH and/or spectral measurements of a mixed solution comprising a mixture of a sample of water from the WF and an added titrant.

In example 54, the subject matter of example 53 may include, wherein the method further comprises detecting leakages in the WF, based on detection of changes in water level over time and exclusion of other causes for water level changes, selected from the exclusion causes of: backwash volume changes, water evaporation.

Although the above description discloses a limited number of exemplary embodiments of the invention, these embodiments should not apply any limitation to the scope of the invention, but rather be considered as exemplifications of some of the manners in which the invention can be implemented.

The method and/or processes described herein may be implemented by any one or more software, and/or hardware, element apparatus, device, mechanism, electronic and/or digital computerized system, unit, processing module, device, machine, engine, etc.

The system, module, unit, device etc. or parts thereof, may be programmed to perform particular functions pursuant to computer readable and executable instructions, rules, conditions etc. from programmable hardware and/or software based execution modules that may implement one or more methods or processes disclosed herein, and therefore can, in effect, be considered as disclosing a "special purpose computer" particular to embodiments of each disclosed method/process.

Additionally or alternatively, the methods and/or processes disclosed herein may be implemented as a computer program that may be tangibly or intangibly embodied by a special purpose computer readable signal medium. A computer readable signal medium may include a propagated data signal with computer readable program code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a non-transitory computer or machine-readable storage device and that can communicate, propagate, or transport a program for use by or in connection with apparatuses, systems, platforms, methods, operations and/or processes discussed herein.

The terms "non-transitory computer-readable storage device" and "non-transitory machine-readable storage device" may also include distribution media, intermediate storage media, execution memory of a computer, and any other medium or device capable of storing for later reading by a computer program implementing embodiments of a method disclosed herein. A computer program product can be deployed to be executed on one computer or on multiple computers at one site or distributed across multiple sites and interconnected by one or more communication networks.

The computer readable and executable instructions may also be loaded onto a computer, other programmable data processing apparatus, or other device to cause a series of operational steps to be performed on the computer, other programmable apparatus or other device to produce a computer implemented process, such that the instructions which execute on the computer, other programmable apparatus, or other device implement the functions/acts specified in the flowchart and/or block diagram block or blocks.

A module, a device, a mechanism, a unit and or a subsystem may each comprise a machine or machines executable instructions (e.g. commands). A module may be embodied by a circuit or a controller programmed to cause the system to implement the method, process and/or operation as disclosed herein. For example, a module may be implemented as a hardware circuit comprising, e.g., custom very large-scale integration (VLSI) circuits or gate arrays, an Application-specific integrated circuit (ASIC), off-the-shelf semiconductors such as logic chips, transistors, and/or other discrete components. A module may also be implemented in programmable hardware devices such as field programmable gate arrays, programmable array logic, programmable logic devices and/or the like.

In the above disclosure, unless otherwise stated, terms such as "substantially", "about", approximately, etc., that specify a condition or relationship characterizing a feature or features of an embodiment of the invention, are to be understood to mean that the condition or characteristic is defined to within tolerances that are acceptable for operation of the embodiment for an application for which it is intended.

It is important to note that the methods/processes and/or systems/devices/subsystems/apparatuses etc., disclosed in the above Specification, are not to be limited strictly to flowcharts and/or diagrams provided in the Drawings. For example, a method may include additional or fewer processes or steps in comparison to what is described in the figures. In addition, embodiments of the method are not necessarily limited to the chronological order as illustrated and described herein.

It is noted that terms such as "processing", "computing", "calculating", "determining", "establishing", "analyzing", "checking", "estimating", "deriving", "selecting", "inferring", identifying", "detecting" and/or the like, may refer to operation(s) and/or process(es) of a computer, a computing platform, a computing system, or other electronic computing device(s), that manipulate and/or transform data represented as physical (e.g., electronic or optical signal) quantities within the computer's registers and/or memories into other data similarly represented as physical quantities within the computer's registers and/or memories or other information storage medium that may store instructions to perform operations and/or processes.

Terms used in the singular shall also include a plural scope, except where expressly otherwise stated or where the context otherwise requires.

In the description and claims of the present application, each of the verbs, "comprise" "include" and "have", and conjugates thereof, are used to indicate that the object or objects of the verb are not necessarily a complete listing of components, elements or parts of the subject or subjects of the verb.

Unless otherwise stated, the use of the expression "and/or" between the last all members of a list of options for selection indicates that a selection of one or more of the listed options is appropriate and may be made i.e. enabling all possible combinations of one or more of the specified options. Further, the use of the expression "and/or" may be used interchangeably with the expressions "at least one of the following", "any one of the following" or "one or more of the following", followed by a listing of the various options.

It is appreciated that certain features of the invention, which are, for clarity, described in the context of separate embodiments or example, may also be provided in combination in a single embodiment. Conversely, various features of the invention, which are, for brevity, described in the context of a single embodiment, example and/or option, may also be provided separately or in any suitable sub-combination or as suitable in any other described embodiment, example or option of the invention. Certain features described in the context of various embodiments, examples and/or optional implementation are not to be considered essential features of those embodiments, unless the embodiment, example and/or optional implementation is inoperative without those elements.

It is noted that the terms "in some embodiments", "according to some embodiments", "according to some embodiments of the invention", "for example", "e.g.", "for instance" and "optionally" may herein be used interchangeably.

The number of elements shown in the Figures should by no means be construed as limiting and is for illustrative purposes only.

It is noted that the terms "operable to" can encompass the meaning of the term "modified or configured to". In other words, a machine "operable to" perform a task can in some embodiments, embrace a mere capability (e.g., "modified") to perform the function and, in some other embodiments, a machine that is actually made (e.g., "configured") to perform the function.

The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals there between.

## Claims

1. An apparatus [1000, 2000] for measuring characteristics of a water facility (WF) [71], the WF comprising at least a piping setup, the apparatus [10000] being **characterized by** comprising at least:

    - a flow control unit [1200, 2200] comprising at least:

        multiple piping inlets and outlets [1271-1275] ; and
        multiple controllable valves [1251-1254, S1-S4];

    - a detection unit [1100, 2100] comprising at least one sensor [1111, 1111', 1170, 1101, 1102, 2111, 2101, P1, P2]; and
    - a main processing and control unit (MPCU) [1800,2001], operatively associated with the flow control unit [1200, 2200] and with the detection unit [1100, 2100],
    wherein the MPCU [1800] is configured to receive and analyze sensor data from the at least one sensor of the detection unit [1100] and to control operation of the controllable valves [1251-1254, S1-S4], for applying an action by selection of different valves states combinations (VSCs), each VSC being associated with a different location and/or a different state of the WF and/or water thereof.

2. The apparatus [1000, 2000] of claim 1, wherein at least some of the controllable valves [1251-1254, S1-S4] are deployed at different inlets or outlets of the multiple inlets and outlets, such as to control water flow to and from different parts of the WF, located at different locations of the WF piping setup [60], while additional controllable valves are configured and located to control water flow to and from different parts of the apparatus [1000, 2000], and/or

    wherein the at least one sensor is selected from: pressure sensor [1170, P1, P2], optical sensor [1111, 1111', 2111], spectroscopic sensor [1111, 1111', 2111], spectrometer, thermometer, acoustic receiver and/or transducer 1101, 1102], and/or
    wherein the detection unit [1100, 2100] further comprises a sampling module [1150, 2150] having a water sample holder [1151, 2151] that is configured for containing a water sample from the WF [71] therein and for filling and drainage of water samples into and from the water sample holder [1151,2151] for sampling water samples from different locations of the WF by controlling at least some of the controllable valves [1251-1254, S1-S4], and/or
    wherein at least one sensor of the at least one sensor of the detection unit [1100, 2100] is positioned and configured for sensing one or more characteristics of the water sample in the water sample holder [1151, 2151], and/or
    wherein the detection unit further comprises at least one optical module [ configured at least for illuminating the sample holder and a water sample contained therein and one or more optical sensors for optically sensing characteristics of the illuminated water sample.

3. The apparatus [1000, 2000] of any one or more of claims 1 to 2, wherein the detection unit [1100, 2100] and/or the MPCU [1800, 2001] are configured for sensing and/or deducing one or more of: spectral characteristics of the water sample, temperature of the water sample, turbidity level of the water sample; hardness level of the water sample, concentration and/or quantity of substances in the water sample.

4. The apparatus [1000, 2000] of claim 3, wherein the at least one optical module [1110, 1120, 1130, 1110', 2120] comprises:

    ● at least one light source [2120] for irradiating the water sample; and
    ● at least one optical detector [1111, 1111', 2111], configured to optically detect characteristics of the water sample in the sampling module, by detection of characteristics of output light emanating from the water sample.

5. The apparatus [1000, 2000] of claim 4, wherein the at least one optical detector [1111, 1111', 2111] comprises at least one of: a spectral optical detector, a spectrometer, a photodetector, a photodiode, a charged coupled device (CCD) camera, a red-green-blue (RGB) sensor.

6. The apparatus [1000, 2000] of claim 5, wherein at least one of the at least one sensor of the detection unit [1100, 2100] is positioned and configured at least to directly detect light emanating from the at least one light source for monitoring spectral detection characteristics of the detection unit.

7. The apparatus [1000, 2000] of any one or more of claims 1 to 6, wherein the different states of the WF measurable by sensing one or more physical characteristics of the WF at different locations thereof, comprise one or more of:

   ● pre and/or post filtering treatment of the WF water;
   ● pre and/or post backwash treatment of the filter of the WF;
   ● pre and/or post degassing of the WF water.
   ● pre and/or post cooling or heating of the WF water;
   ● pre and/or post drainage of the WF water.

8. The apparatus [1000, 2000] of any one or more of claims 1 to 7 is further configured to sample water from the WF and to apply one or more manipulations over the sampled water, wherein the one or more manipulation comprises one or more of:

   applying of one or more chemical treatments of at least one water sample obtained from the WF;
   conducting one or more biomass incubations of at least one water sample obtained from the WF;
   applying of acoustic one or more signals to at least one water sample obtained from the WF;

   ● changing temperature of the water sample;
   ● adding and/or removing one or more substances (such as Chlorine) from the water sample;

   changing pH of the water sample

9. The apparatus [1000, 2000] of any one or more of claims 1 to 8 further comprising one or more of:

   a degasification module [1500, 2500],
   an alkalinity module [1400] for controlling and/or measuring pH level of the water in the WF, and/or converting HOCl to OCl for the purpose of measuring same, and/or for estimating background water spectrum;
   a cooling mechanism [40, 2800] comprising one or more cooling channels enabling channeling of a cooling liquid or gas for cooling parts of the apparatus
   one or more acoustic transducers for applying an acoustic signal to water in a sampling module [1150, 2150] of the apparatus [1000, 2000] having a water sample holder [1151, 2151] for holding therein a sample of the WF water, the one or more acoustic transducers [1101, 1102] being used for detection of one or more characteristics of the WF, the WF water and/or for cleaning of the interior of sample holder.

10. The apparatus [1000, 2000] of claims 1 to 9, wherein the one or more characteristics of the WF and/or water thereof, comprising one or more of: water hardness, concentration and/or quantity of organic and/or chemical substances, WF devices' functionality, WF piping condition, one or more WF pumps and/or filters functionality.

11. The apparatus [1000, 2000] of any one or more of claims 1 to 10, wherein the MPCU [1800, 2001] is further configured to transmit information indicative of measured WF characteristics to one or more remote devices and/or systems via one or more communication links, for enabling the transmitted information to be presented, displayed, printed, indicated, further processed and/or stored by one or more external devices and/or systems.

12. The apparatus [1000, 2000] of any one or more of claims 1 to 11, wherein the MPCU [1800, 2001] is further configured to:

   operate an executable monitoring plan including executable scheduled monitoring actions;
   select detection location "Li" for each monitoring action and an associated valves states combination I (VSCi);

control direction and/or interconnection of water from the selected detection location Li to one or more sensors or a sampling module of the apparatus by controlling the valves according to the selected VSCi;

for each of at least some of WF parts' characteristics: acquire sensor data from the at least one sensor of the apparatus for different locations and/or state of the WF according to the executable monitoring plan;

analyze the acquired sensor data to detect one or more characteristics of the WF, based on the sensor data, using one or more analysis algorithms; and

output information indicative of detected WF characteristics.

**13.** The apparatus [1000, 2000] of any one or more of claims 1 to 12, wherein the WF further comprises at least one of the following WF devices and/or components:

at least one skimmer [73], and wherein the at least one pressure sensor of the apparatus is further used for detection of updated skimmer water level;

at least one pipeline [60, 65, 101,102] of the piping of the WF;

at least one filter [63];

at least one pump [62];

wherein the apparatus [1000, 2000] is further configured to detect and/or determine state of each of the above devices and/or components of the WF, by analyzing sensor data received from corresponding one or more sensors.

**14.** The apparatus [1000, 2000] of claim 13 wherein the one or more corresponding one or more sensors comprises at least one pressure sensor.

**15.** A method for measuring various characteristics of a water facility (WF) [71] and/or of water from the WF, the WF [71] comprising at least a piping setup, the method being **characterized by** comprising at least:

providing the apparatus [1000, 2000] of any one of claims 1 to 14;

operating an executable monitoring plan [21] including executable scheduled monitoring actions, wherein each monitoring action is associated at least with one or more specific valves states combination and with a specific location in the WF and/or with one or more specific actions applicable to water from the WF;

selecting a specific "i" valves state combination (VSCi) [22], according to the currently executed monitoring action and control the valves based on the selected VSCi associated with the specific currently executed monitoring action;

measuring one or more characteristics of water from the WF and/or of the WF [24], for the respective operated executable monitoring plan, based on analysis of sensor data, acquired by the at least one sensor, during the execution of the specific monitoring action; and

outputting information indicative of measured characteristics [25].

**FIG. 1**

EP 4 194 643 A2

FIG. 2A

FIG. 2B

FIG. 3

FIG. 4A

1001

40

1000

FIG. 4C

FIG. 4B

EP 4 194 643 A2

**FIG. 5**

FIG. 6

FIG. 7

List of Cases And Sequences:

| | | Init State | Stop Flow For Measuring | With Debubler — Activity Cooling | | Natural Cooling | Flow Measuring/ Chlorinator Testing/Filter Clogging | Alkalinity Dosing |
|---|---|---|---|---|---|---|---|---|
| | | | | Automatic Empty | Activity Cooling | Automatic Empty | | |
| Solenoid 1 (2W) | 12V | Closed | Closed | Closed | Closed | Closed | Open | Closed |
| Solenoid 2 (2W) | 12V | Open | Closed | Closed | Open | Closed | Closed | Open |
| Solenoid 3 (3W) | 12V | Open | Closed | Closed | Open | Closed | Open | Open |
| Solenoid 4 (2W) | 12V | Closed | Closed | Closed | Closed | Open | Closed | Closed |
| Pressure Transduser PT5 | 4-20mA | • • • | • • • | • • • | • • • | • • • | • • • | • • • |
| Pressure Transduser PT4 | 4-20mA | • • • | • • • | • • • | • • • | • • • | • • • | • • • |
| Conductivity/ Temp Sensor | | • • • | • • • | <=4 Deg | • • • | <=4 Deg | • • • | • • • |
| Peristaltic Pump | 12V | OFF | OFF | OFF | OFF | OFF | OFF | 10(S) |
| Diaphragm Pump | 12V | OFF | OFF | ON (1min) | ON | OFF | OFF | OFF |
| Air Temp Sensor | | | | | >=45 Deg | | | |

FIG. 8

EP 4 194 643 A2

FIG. 9

EP 4 194 643 A2

FIG. 10A

EP 4 194 643 A2

FIG. 10B

Time

Flow [L/m]

Pressure [bar]

Sensor PT 7 ----
Sensor PT 5 ——

Start Pressure Difference Between
PT5 and PT7 (clogging and beginning)

36

FIG. 10C

FIG. 11

Selecting/executing a monitoring plan including executable scheduled actions — 21

Selecting a specific "i" valve state combination (VSCi), according to the currently executed monitoring action of the plan and control the valves based on the associated selected VSCi — 22

operating one or more manipulations to the water of the WF (e.g., by first acquiring a water sample and applying manipulations to the water sample), according to the currently operated "i" monitoring action, if the specific currently operated monitoring action is associated with one or more manipulations — 23

measuring one or more characteristics of the WF, for the respective operated executable monitoring plan, based analysis of sensor data, acquired by the at least one sensor, during the execution of the specific monitoring action — 24

outputting information indicative of measured WF characteristics — 25

FIG. 12